# EUROPEAN PATENT APPLICATION

(11) **EP 4 327 811 A1**
(43) Date of publication of application: **28.02.2024**
(21) Application number: 22791959.4
(22) Date of filing: 14.04.2022
(51) Int. Cl.: A61K 31/496, A61P 35/00, A23L 33/10, A61K 31/352, A61P 43/00, A61P 11/00, A61K 45/06

(54) **ANTICANCER COMPOSITION INDUCING CELL SENESCENCE AND CELL DEATH**

(30) Priority: 20.04.2021 KR 20210050983; 31.05.2021 KR 20210069844; 16.07.2021 KR 20210093288; 23.12.2021 KR 20210186561; 17.03.2022 KR 20220033299
(71) Applicant: Osteoneurogen Inc., Seoul 08507 (KR)
(72) Inventor: YOUN, Byung Soo, Seoul 08210 (KR); MEANG, Moon Kee, Seoul 03666 (KR); KIM, Saes Byeol, Seoul 02511 (KR); KIM, Ik Hwan, Seoul 04587 (KR); KIM, Han Soo, Seoul 08735 (KR)
(74) Representative: Petty, Catrin Helen
(86) International application number: PCT/KR2022/005410
(87) International publication number: WO 2022/225259

(57) **Abstract**

The present invention relates to an anticancer composition containing as an active ingredient a compound inducing cell senescence and apoptosis. The compound of the present invention can discriminate cancer cells and normal cells and induce both cell senescence and apoptosis in a cancer cell-specific manner. Thus, the compound does not need the attachment of any cancer cell-specific targeting substance thereto, can minimize the frequent adverse effects caused by the attack of anticancer agents on normal cells, and exhibits a potent anticancer effect on the basis of the aforementioned dual action.

## Description

### [Technical Field]

The present invention relates to an anticancer composition containing a compound that induces cell senescence and apoptosis as an active ingredient, and more specifically to an antitumor or anticancer composition that exhibits an antitumor or anticancer effect by containing a compound represented by Chemical Formula 1 or a salt thereof as an active ingredient and inducing cell senescence and apoptosis specifically in tumor or cancer cells.

### [Background Art]

A tumor refers to an abnormally grown mass due to the autonomous overgrowth of body tissues, and it can be classified into benign tumors and malignant tumors. While benign tumors have a relatively slow growth rate and do not metastasize (the tumor moves away from the original location), malignant tumors infiltrate the surrounding tissues to grow rapidly and spread or metastasize to various parts of the body, thereby threatening lives. Therefore, malignant tumor can be regarded as the same meaning as cancer.

A cell, which is the smallest unit of the body, normally divides and grows by the control function of the cell itself, and when its lifespan expires or is damaged, it dies (dying and disappearing) and maintains the balance of the overall number. However, if there is a problem with the control function of these cells themselves due to various causes, abnormal cells that normally should be killed proliferate excessively, thereby destroying and transforming the existing structure, and this condition can be defined as cancer.

Small molecules targeting the cell cycle have been considered to cause cancer because of undesirable medical risks, such as the systemic inhibition of stem cell differentiation or the deterioration of homeostatic immunity. However, natural drug scaffolds such as chromone-scaffolds, which are mainly found in flavones or isoflavones, have the advantage of being developed for medical use because they have been proven to be mostly safe as natural products. Actually, SB203580, which is one of the chromone scaffold derivatives, has been reported to have anticancer activity (Yan W et al., Toxicol Lett 2016; 259: 28-34. DOI: 10.1016/j.toxlet.2016.07.591).

Flavones are members of the polyphenol family, and a group of more than 10,000 compounds was found exclusively in the plant kingdom alone. In general, these phytochemicals function to protect plants from radiation damage. Flavones have long been used to treat inflammatory diseases such as arthritis and asthma due to their antioxidant or anti-inflammatory potential, and chromone (1,4-benzopyrone-4-one) is a central chemical support that makes up flavones and isoflavones.

The inventors of the present invention recently reported that eupatilin, which is a chromone scaffold-containing compound from *Artemisia* species, and the compound of the present invention (Korean Registered Patent No. 10-1871166) inhibit fibrosis by degrading the actin polymer, and as a result, it has been confirmed that epithelial-mesenchymal metastasis (EMT) is inhibited (Kim HS et al., bioRxiv 2020; 770404. DOI: 10.1101/770404).

In this regard, while the inventors of the present invention were conducting further studies, it was confirmed that the compound of the present invention is a potent inducer of replicative senescence (RS) and oncogene-induced senescence (OIS) in several aggressive cancer cell lines that inhibits uncontrolled proliferation of cancer cells by such cellular senescence mechanisms and induces escape from tumorization through the reversal of the NAD/NADH ratio by NAD biosynthesis and upregulation of ROS, and in addition, it can function as a potent therapeutic agent for cancer treatment through a series of actions that induce apoptosis, thereby completing the present invention.

### [Disclosure]

### [Technical Problem]

The present invention provides the anti-tumor and/or anti-cancer use of a chromone scaffold derivative that simultaneously induces cell senescence and apoptosis.

### [Technical Solution]

In order to achieve the above objects, the present invention provides a pharmaceutical composition for preventing or treating tumor or cancer disease, including a compound represented by Chemical Formula 1 below or a salt thereof as an active ingredient: 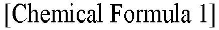 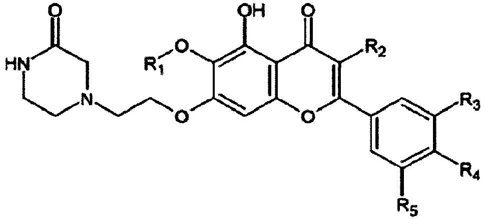
wherein in Chemical Formula 1 above,
R₁ is a C₁₋₅ alkyl, a C₅₋₆ cyclic alkyl, a C₅₋₆ cyclic alkyl containing at least one heteroatom of O or N, a C₆₋₁₂ aryl or a C₅₋₆ heteroaryl containing at least one heteroatom of O or N,
wherein R₂ is hydrogen, ethyl, acetyl, acetoxy, carboxy, benzoyloxy or 3,4,5-trihydroxybenzoyloxy,
wherein R₃ is hydrogen, hydroxy, methyl, methoxy, acetoxy, carboxy or benzoyloxy,
wherein R₄ is hydrogen, hydroxy, methyl, methoxy, acetoxy, carboxy or benzoyloxy, and
wherein R₅ is hydrogen, hydroxy, methyl, methoxy, acetoxy, carboxy or benzoyloxy.

In addition, the present invention provides a quasi-drug composition for preventing or ameliorating tumor or cancer disease, including a compound represented by Chemical Formula 1 below or a salt thereof as an active ingredient: 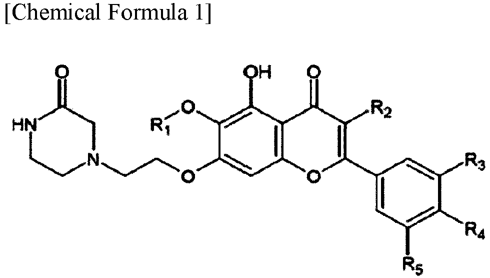
wherein in Chemical Formula 1 above,
R₁ is a C₁₋₅ alkyl, a C₅₋₆ cyclic alkyl, a C₅₋₆ cyclic alkyl containing at least one heteroatom of O or N, a C₆₋₁₂ aryl or a C₅₋₆ heteroaryl containing at least one heteroatom of O or N,
wherein R₂ is hydrogen, ethyl, acetyl, acetoxy, carboxy, benzoyloxy or 3,4,5-trihydroxybenzoyloxy,
wherein R₃ is hydrogen, hydroxy, methyl, methoxy, acetoxy, carboxy or benzoyloxy,
wherein R₄ is hydrogen, hydroxy, methyl, methoxy, acetoxy, carboxy or benzoyloxy, and
wherein R₅ is hydrogen, hydroxy, methyl, methoxy, acetoxy, carboxy or benzoyloxy.

In addition, the present invention provides a food composition for preventing or ameliorating tumor or cancer disease, including a compound represented by Chemical Formula 1 below or a salt thereof as an active ingredient: 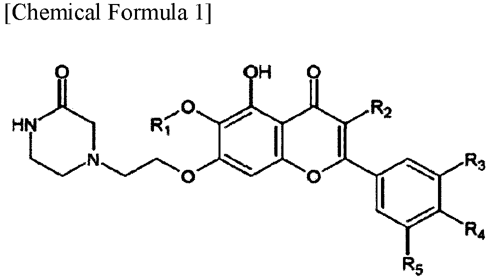
wherein in Chemical Formula 1 above,
R₁ is a C₁₋₅ alkyl, a C₅₋₆ cyclic alkyl, a C₅₋₆ cyclic alkyl containing at least one heteroatom of O or N, a C₆₋₁₂ aryl or a C₅₋₆ heteroaryl containing at least one heteroatom of O or N,
wherein R₂ is hydrogen, ethyl, acetyl, acetoxy, carboxy, benzoyloxy or 3,4,5-trihydroxybenzoyloxy,
wherein R₃ is hydrogen, hydroxy, methyl, methoxy, acetoxy, carboxy or benzoyloxy,
wherein R₄ is hydrogen, hydroxy, methyl, methoxy, acetoxy, carboxy or benzoyloxy, and
wherein R₅ is hydrogen, hydroxy, methyl, methoxy, acetoxy, carboxy or benzoyloxy.

In addition, the present invention provides a method for preventing or treating tumor or cancer disease, including the step of administering a compound represented by Chemical Formula 1 below or a salt thereof to a subject in need thereof: 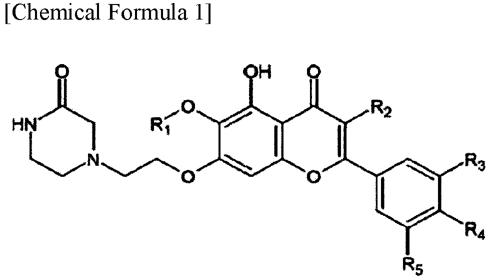
wherein in Chemical Formula 1 above,
R₁ is a C₁₋₅ alkyl, a C₅₋₆ cyclic alkyl, a C₅₋₆ cyclic alkyl containing at least one heteroatom of O or N, a C₆₋₁₂ aryl or a C₅₋₆ heteroaryl containing at least one heteroatom of O or N,
wherein R₂ is hydrogen, ethyl, acetyl, acetoxy, carboxy, benzoyloxy or 3,4,5-trihydroxybenzoyloxy,
wherein R₃ is hydrogen, hydroxy, methyl, methoxy, acetoxy, carboxy or benzoyloxy,
wherein R₄ is hydrogen, hydroxy, methyl, methoxy, acetoxy, carboxy or benzoyloxy, and
wherein R₅ is hydrogen, hydroxy, methyl, methoxy, acetoxy, carboxy or benzoyloxy.

In addition, the present invention provides the use of a compound represented by Chemical Formula 1 below or a salt thereof in preventing or ameliorating tumor or cancer disease: 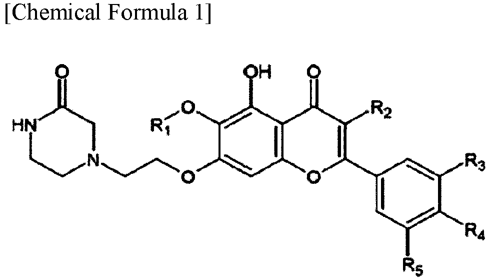
wherein in Chemical Formula 1 above,
R₁ is a C₁₋₅ alkyl, a C₅₋₆ cyclic alkyl, a C₅₋₆ cyclic alkyl containing at least one heteroatom of O or N, a C₆₋₁₂ aryl or a C₅₋₆ heteroaryl containing at least one heteroatom of O or N,
wherein R₂ is hydrogen, ethyl, acetyl, acetoxy, carboxy, benzoyloxy or 3,4,5-trihydroxybenzoyloxy,
wherein R₃ is hydrogen, hydroxy, methyl, methoxy, acetoxy, carboxy or benzoyloxy,
wherein R₄ is hydrogen, hydroxy, methyl, methoxy, acetoxy, carboxy or benzoyloxy, and
wherein R₅ is hydrogen, hydroxy, methyl, methoxy, acetoxy, carboxy or benzoyloxy.

In addition, the present invention provides the use of a compound represented by Chemical Formula 1 below or a salt thereof in the preparation of a drug for preventing or treating tumor or cancer disease: 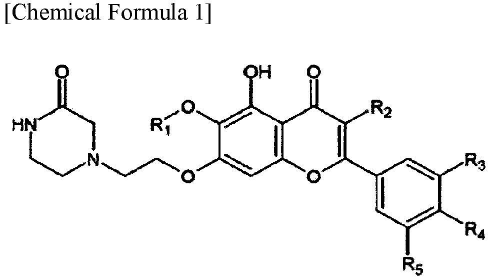
wherein in Chemical Formula 1 above,
R₁ is a C₁₋₅ alkyl, a C₅₋₆ cyclic alkyl, a C₅₋₆ cyclic alkyl containing at least one heteroatom of O or N, a C₆₋₁₂ aryl or a C₅₋₆ heteroaryl containing at least one heteroatom of O or N,
wherein R₂ is hydrogen, ethyl, acetyl, acetoxy, carboxy, benzoyloxy or 3,4,5-trihydroxybenzoyloxy,
wherein R₃ is hydrogen, hydroxy, methyl, methoxy, acetoxy, carboxy or benzoyloxy,
wherein R₄ is hydrogen, hydroxy, methyl, methoxy, acetoxy, carboxy or benzoyloxy, and
wherein R₅ is hydrogen, hydroxy, methyl, methoxy, acetoxy, carboxy or benzoyloxy.

In the present invention, R₁ may be methyl, ethyl, cyclopentyl, cyclohexyl or phenyl.

In the present invention, R₁ may be methyl,
wherein R₂ may be hydrogen,
wherein R₃ may be hydrogen, hydroxy or methoxy,
wherein R₄ may be hydroxy or methoxy, and
wherein R₅ may be hydrogen, hydroxy or methoxy.

In the present invention, the compound may be represented by any one of Chemical Formulas 2 to 5 below:

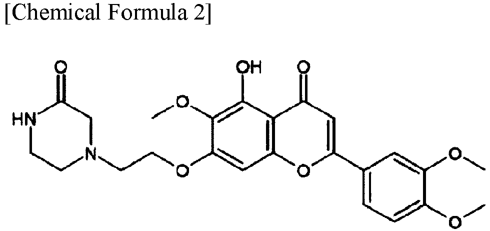

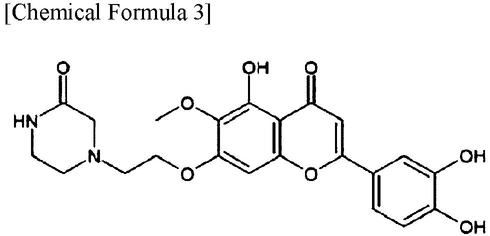

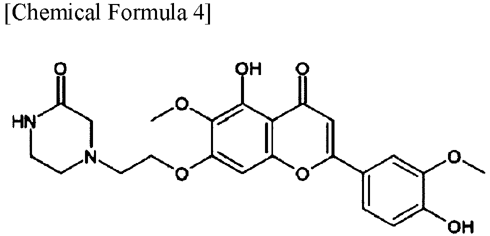

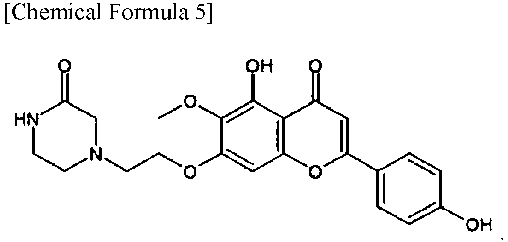

In the present invention, the cancer may be blood cancer or solid cancer.

In the present invention, the blood cancer may be leukemia, malignant lymphoma, multiple myeloma or aplastic anemia.

In the present invention, the solid cancer may be brain tumor, benign astrocytoma, malignant astrocytoma, pituitary adenoma, meningioma, brain lymphoma, oligodendroglioma, craniopharyngioma, ependymoma, brainstem tumor, head and neck tumor, laryngeal cancer, oropharyngeal cancer, nasal cancer, nasopharyngeal cancer, salivary gland cancer, hypopharyngeal cancer, thyroid cancer, oral cancer, chest tumor, small cell lung cancer, non-small cell lung cancer, thymus cancer, mediastinal tumor, esophageal cancer, breast cancer, male breast cancer, abdominal tumor, stomach cancer, liver cancer, gallbladder cancer, biliary tract cancer, pancreatic cancer, small intestine cancer, colon cancer, anal cancer, bladder cancer, kidney cancer, male genital tumor, penile cancer, prostate cancer, female genital tumor, cervical cancer, endometrial cancer, ovarian cancer, uterine sarcoma, vaginal cancer, female external genital cancer, female urethral cancer or skin cancer.

In the present invention, the compound may exhibit antitumor or anticancer activity by at least one action selected from the group consisting of:
(i) inhibition of tumor or cancer cell-specific proliferation by the induction of replicative senescence (RS) and/or oncogene-induced senescence (OIS);
(ii) reversal of the tumor or cancer cell NAD/NADH ratio;
(iii) upregulation of tumor or cancer cell ROS; and
(iv) inducing tumor or cancer cell-specific apoptosis.

### [Advantageous Effects]

Since the compound of the present invention can discriminate cancer cells from normal cells and induce cancer cell-specific cell senescence and apoptosis at the same time, there is no need to attach a cancer cell-specific targeting material. In addition, it is possible to minimize side effects that often occur when anticancer drugs attack normal cells, and it has a characteristic that can exert a strong anticancer effect due to the aforementioned dual action.

### [Description of Drawings]

FIG. 1 is a comparison result of the cell senescence-inducing effect in A549 cells according to the treatment of the compound of Chemical Formula 2 (ONG41008), eupatylin (ONG21002) and other chromone scaffold-containing derivatives according to an exemplary embodiment of the present invention.
FIG. 2 is a result of observing changes in the expression level and intracellular position of (a) p53, (b) p21 and (c) p16 in A549 cells after 24 hours of treatment by concentration of the compound of Chemical Formula 2 (ONG41008) according to an exemplary embodiment of the present invention.
FIG. 3 is a result of observing changes in the expression level and intracellular position of p16 in A549 cells after 72 hours of treatment with the compound of Chemical Formula 2 (ONG41008) (10 µM) according to an exemplary embodiment of the present invention.
FIG. 4a is a result of observing changes in the expression level of p21, p16, p53 and p-p53 after 24 hours of treatment by concentration of the compound of Chemical Formula 2 (ONG41008) according to an exemplary embodiment of the present invention by Western blot (the location of the corresponding protein band is indicated in yellow), and FIG. 4b is a result of observing the cell change according to the treatment of the compound of Chemical Formula 2 (ONG41008) according to an exemplary embodiment of the present invention after inducing the proliferation of A549 cells by TGFβ.
FIG. 5 is a result of confirming the degree of cell senescence and multinucleation of A549 cells according to the lapse of time ((a) 24 hours, (b) 48 hours, (c) 72 hours) after treatment with the compound of Chemical Formula 2 (ONG41008) according to an exemplary embodiment of the present invention.
FIG. 6 is a result of observing the mitochondrial membrane potential and confirming the cell proliferation inhibitory effect by treating the compound of Chemical Formula 2 (ONG41008) or SAHA according to an exemplary embodiment of the present invention.
FIG. 7a is a result of confirming the effect of the compound of Chemical Formula 2 (ONG41008) according to an exemplary embodiment of the present invention on the cell cycle of A549 cells by Western blot.
FIG. 7b is a result of treating the compound of Chemical Formula 2 (ONG41008) according to an exemplary embodiment of the present invention by concentration and analyzing how the compound affects he cell cycle of A549 cells by PI staining.
FIG. 7c is a result of treating the compound of Chemical Formula 2 (ONG41008) or SAHA according to an exemplary embodiment of the present invention and confirming the effect on the NAD/NADH ratio of A549 cells.
FIG. 7d is a result of confirming the effect of the compound of Chemical Formula 2 (ONG41008) according to an exemplary embodiment of the present invention on ROS generation in A549 cells.
FIG. 8 is a result of confirming the apoptosis of A549 cells according to the treatment of the compound of Chemical Formula 2 (ONG41008) according to an exemplary embodiment of the present invention.
FIG. 9 is a result of confirming the cell proliferation inhibitory effect of H358 cells by treatment with the compound of Chemical Formula 2 (ONG41008) or paclitaxel according to an exemplary embodiment of the present invention by CCK-8 analysis.
FIG. 10 is an electron micrograph showing the mitotic disruption of H358 cells according to the treatment of the compound of Chemical Formula 2 (ONG41008) according to an exemplary embodiment of the present invention.
FIG. 11 is a result of confirming the multinucleation of H1299 cells according to the concentration-specific treatment of the compound of Chemical Formula 2 (ONG41008) according to an exemplary embodiment of the present invention.
FIG. 12 is a result of confirming the cell proliferation inhibitory effect of PANC1 cells according to the treatment the compound of Chemical Formula 2 (ONG41008) or paclitaxel according to an exemplary embodiment of the present invention by CCK-8 analysis.
FIG. 13 is a result confirming the multinucleation of PANC1 cells according to the treatment of the compound of Chemical Formula 2 (ONG41008) according to an exemplary embodiment of the present invention.
FIG. 14 is a result of confirming the cell proliferation inhibitory effect of MCF7 cells according to the treatment of a compound of Chemical Formula (ONG41008) or SAHA according to an exemplary embodiment of the present invention by CCK-8 analysis.
FIG. 15 is a result of confirming the multinucleation of MCF7 cells according to the treatment of the compound of Chemical Formula 2 (ONG41008) according to an exemplary embodiment of the present invention.
FIG. 16 is a result of confirming the cell proliferation inhibitory effect of PC3 cell according to the treatment of the compound of Chemical Formula 2 (ONG41008) or SAHA according to an exemplary embodiment of the present invention by CCK-8 analysis.
FIG. 17 is a result of confirming the apoptosis of PC3 cells according to the treatment of the compound of Chemical Formula 2 (ONG41008) or SAHA according to an exemplary embodiment of the present invention by Caspase 3/7 analysis.
FIG. 18 is a result of confirming the multinucleation of PC3 cells according to the treatment of the compound of Chemical Formula 2 (ONG41008) according to an exemplary embodiment of the present invention.
FIG. 19 is a result of treating normal human lung fibroblasts with the compound of Chemical Formula 2 (ONG41008), nintedanib or SAHA according to an exemplary embodiment of the present invention, and comparing the cell proliferation inhibitory effects by CCK-8 assay.
FIG. 20 is a result of comparing whether apoptosis is induced by treating normal human lung fibroblasts with the compound of Chemical Formula 2 (ONG41008), nintedanib or SAHA according to an exemplary embodiment of the present invention.
FIG. 21 is a summary of the mechanism of action of inducing the anticancer (or antitumor) effect of the compound of Chemical Formula 2 (ONG41008) and eupatylline (ONG21002) according to an exemplary embodiment of the present invention in comparison with other chromone scaffold derivatives.
FIG. 22 is a schematic diagram showing that the compound of the present invention can exert the effect induced by the combined administration of a conventional anticancer agent and a chromone scaffold derivative by administration alone.

### [Modes of the Invention]

Unless defined otherwise, all technical and scientific terms used herein have the same meanings as commonly understood by one of ordinary skill in the art to which this invention pertains. In general, the nomenclature used herein is those well-known and commonly used in the art.

*In vivo,* the cell cycle is delicately regulated such that proliferation, differentiation and apoptosis can be coordinated, and when the disruption of these sophisticated regulatory functions occurs, it can lead to serious disease, and particularly, uncontrolled cell proliferation leads to diseases that are both fatal and intractable to the human body, such as cancer. Therefore, in order to treat cancer, it is necessary to inhibit cell proliferation, and anticancer agents exhibiting a cell proliferation inhibitory function generate many side effects along with the cell proliferation inhibitory function.

In the present invention, it was confirmed that the compound of the present invention, which is one of the chromone scaffold derivatives, can resolve the uncontrolled proliferation state of various cancer cell lines by cell senescence (*i.e.,* senescence caused by replication senescence and oncogene induction). Interestingly, it was confirmed that the compound of the present invention does not induce cell senescence in normal cells and does not inhibit normal cell proliferation, and thus, it has the advantage in that there is no need to separately attach a substance targeting cancer cells as in general anticancer drugs. In addition, when the compound of the present invention was orally administered to mice or rats, it was further verified that no pathological abnormality or toxicity was observed (Preclinical Study Number: U-18156, Administration Dose 1g), and therefore, it was found that the compound of the present invention can safely exert anticancer effects without side effects.

Therefore, in a first aspect, the present invention relates a pharmaceutical composition for preventing or treating tumor or cancer disease, including a compound represented by Chemical Formula 1 below or a salt thereof as an active ingredient: 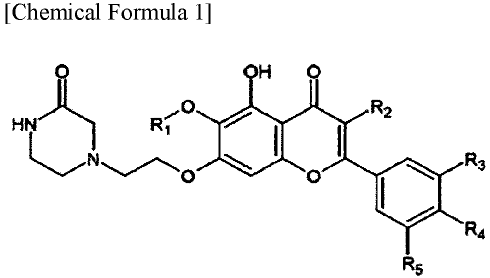
wherein in Chemical Formula 1 above,
R₁ is a C₁₋₅ alkyl, a C₅₋₆ cyclic alkyl, a C₅₋₆ cyclic alkyl containing at least one heteroatom of O or N, a C₆₋₁₂ aryl or a C₅₋₆ heteroaryl containing at least one heteroatom of O or N,
wherein R₂ is hydrogen, ethyl, acetyl, acetoxy, carboxy, benzoyloxy or 3,4,5-trihydroxybenzoyloxy,
wherein R₃ is hydrogen, hydroxy, methyl, methoxy, acetoxy, carboxy or benzoyloxy,
wherein R₄ is hydrogen, hydroxy, methyl, methoxy, acetoxy, carboxy or benzoyloxy, and
wherein R₅ is hydrogen, hydroxy, methyl, methoxy, acetoxy, carboxy or benzoyloxy.

Further, in a second aspect, the present invention relates to a quasi-drug composition for preventing or ameliorating tumor or cancer disease, including a compound represented by Chemical Formula 1 below or a salt thereof as an active ingredient: 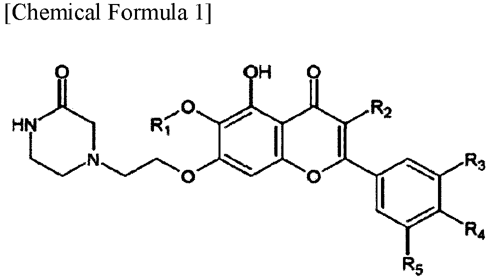
wherein in Chemical Formula 1 above,
R₁ is a C₁₋₅ alkyl, a C₅₋₆ cyclic alkyl, a C₅₋₆ cyclic alkyl containing at least one heteroatom of O or N, a C₆₋₁₂ aryl or a C₅₋₆ heteroaryl containing at least one heteroatom of O or N,
wherein R₂ is hydrogen, ethyl, acetyl, acetoxy, carboxy, benzoyloxy or 3,4,5-trihydroxybenzoyloxy,
wherein R₃ is hydrogen, hydroxy, methyl, methoxy, acetoxy, carboxy or benzoyloxy,
wherein R₄ is hydrogen, hydroxy, methyl, methoxy, acetoxy, carboxy or benzoyloxy, and
wherein R₅ is hydrogen, hydroxy, methyl, methoxy, acetoxy, carboxy or benzoyloxy.

Further, in a third aspect, the present invention relates to a food composition for preventing or ameliorating tumor or cancer disease, including a compound represented by Chemical Formula 1 below or a salt thereof as an active ingredient: 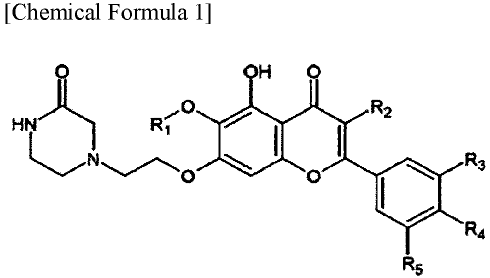
wherein in Chemical Formula 1 above,
R₁ is a C₁₋₅ alkyl, a C₅₋₆ cyclic alkyl, a C₅₋₆ cyclic alkyl containing at least one heteroatom of O or N, a C₆₋₁₂ aryl or a C₅₋₆ heteroaryl containing at least one heteroatom of O or N,
wherein R₂ is hydrogen, ethyl, acetyl, acetoxy, carboxy, benzoyloxy or 3,4,5-trihydroxybenzoyloxy,
wherein R₃ is hydrogen, hydroxy, methyl, methoxy, acetoxy, carboxy or benzoyloxy,
wherein R₄ is hydrogen, hydroxy, methyl, methoxy, acetoxy, carboxy or benzoyloxy, and
wherein R₅ is hydrogen, hydroxy, methyl, methoxy, acetoxy, carboxy or benzoyloxy.

Further, in a fourth aspect, the present invention relates to a method for preventing or treating tumor or cancer disease, including the step of administering a compound represented by Chemical Formula 1 below or a salt thereof to a subject in need thereof: 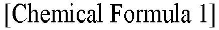 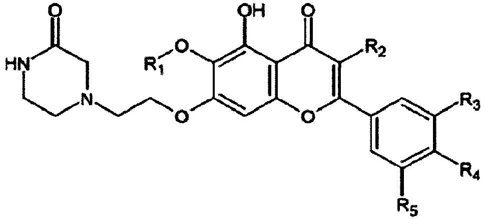
wherein in Chemical Formula 1 above,
R₁ is a C₁₋₅ alkyl, a C₅₋₆ cyclic alkyl, a C₅₋₆ cyclic alkyl containing at least one heteroatom of O or N, a C₆₋₁₂ aryl or a C₅₋₆ heteroaryl containing at least one heteroatom of O or N,
wherein R₂ is hydrogen, ethyl, acetyl, acetoxy, carboxy, benzoyloxy or 3,4,5-trihydroxybenzoyloxy,
wherein R₃ is hydrogen, hydroxy, methyl, methoxy, acetoxy, carboxy or benzoyloxy,
wherein R₄ is hydrogen, hydroxy, methyl, methoxy, acetoxy, carboxy or benzoyloxy, and
wherein R₅ is hydrogen, hydroxy, methyl, methoxy, acetoxy, carboxy or benzoyloxy.

Further, in a fifth aspect, the present invention relates to the use of a compound represented by Chemical Formula 1 below or a salt thereof in preventing or ameliorating tumor or cancer disease: 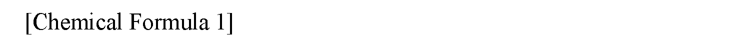 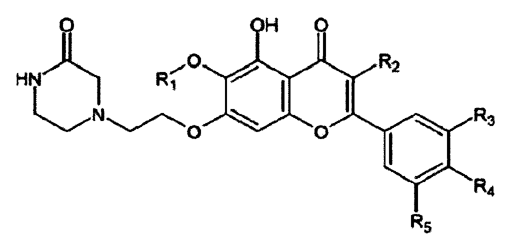
wherein in Chemical Formula 1 above,
R₁ is a C₁₋₅ alkyl, a C₅₋₆ cyclic alkyl, a C₅₋₆ cyclic alkyl containing at least one heteroatom of O or N, a C₆₋₁₂ aryl or a C₅₋₆ heteroaryl containing at least one heteroatom of O or N,
wherein R₂ is hydrogen, ethyl, acetyl, acetoxy, carboxy, benzoyloxy or 3,4,5-trihydroxybenzoyloxy,
wherein R₃ is hydrogen, hydroxy, methyl, methoxy, acetoxy, carboxy or benzoyloxy,
wherein R₄ is hydrogen, hydroxy, methyl, methoxy, acetoxy, carboxy or benzoyloxy, and
wherein R₅ is hydrogen, hydroxy, methyl, methoxy, acetoxy, carboxy or benzoyloxy.

Further, in a sixth aspect, the present invention relates to the use of a compound represented by Chemical Formula 1 below or a salt thereof in the preparation of a drug for preventing or treating tumor or cancer disease: 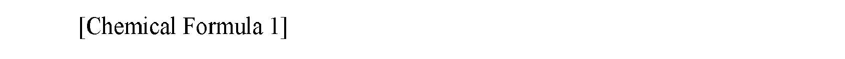 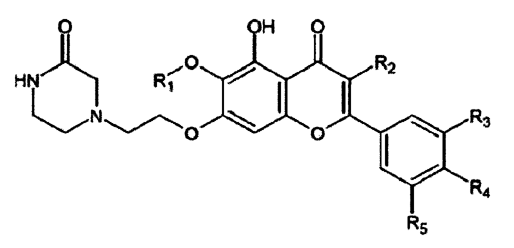
wherein in Chemical Formula 1 above,
R₁ is a C₁₋₅ alkyl, a C₅₋₆ cyclic alkyl, a C₅₋₆ cyclic alkyl containing at least one heteroatom of O or N, a C₆₋₁₂ aryl or a C₅₋₆ heteroaryl containing at least one heteroatom of O or N,
wherein R₂ is hydrogen, ethyl, acetyl, acetoxy, carboxy, benzoyloxy or 3,4,5-trihydroxybenzoyloxy,
wherein R₃ is hydrogen, hydroxy, methyl, methoxy, acetoxy, carboxy or benzoyloxy,
wherein R₄ is hydrogen, hydroxy, methyl, methoxy, acetoxy, carboxy or benzoyloxy, and
wherein R₅ is hydrogen, hydroxy, methyl, methoxy, acetoxy, carboxy or benzoyloxy.

In any one aspect of the present invention, R₁ may be methyl, ethyl, cyclopentyl, cyclohexyl or phenyl.

In any one aspect of the present invention R₁ may be methyl,
wherein R₂ may be hydrogen,
wherein R₃ may be hydrogen, hydroxy or methoxy,
wherein R₄ may be hydroxy or methoxy, and
wherein R₅ may be hydrogen, hydroxy or methoxy.

In any one aspect of the present invention the compound may be represented by any one of Chemical Formulas 2 to 5 below:

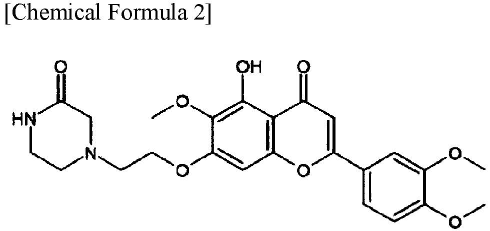

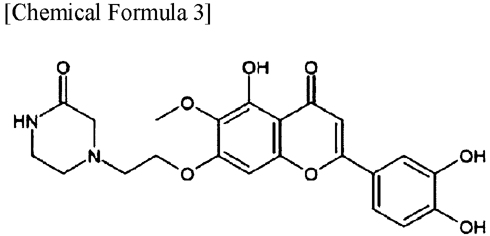

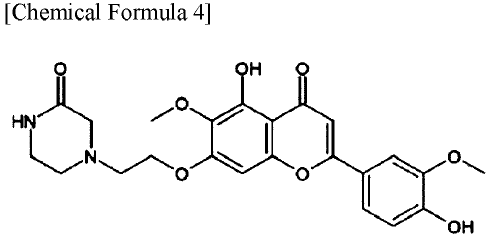

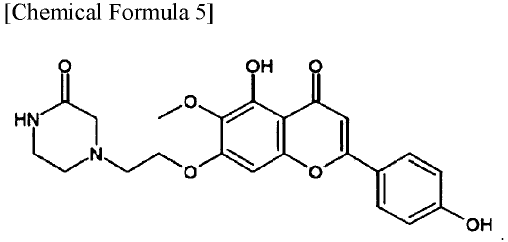

In any one aspect of the present invention the cancer may be blood cancer or solid cancer.

In any one aspect of the present invention the blood cancer may be leukemia, malignant lymphoma, multiple myeloma or aplastic anemia.

In any one aspect of the present invention the solid cancer may be brain tumor, benign astrocytoma, malignant astrocytoma, pituitary adenoma, meningioma, brain lymphoma, oligodendroglioma, craniopharyngioma, ependymoma, brainstem tumor, head and neck tumor, laryngeal cancer, oropharyngeal cancer, nasal cancer, nasopharyngeal cancer, salivary gland cancer, hypopharyngeal cancer, thyroid cancer, oral cancer, chest tumor, small cell lung cancer, non-small cell lung cancer, thymus cancer, mediastinal tumor, esophageal cancer, breast cancer, male breast cancer, abdominal tumor, stomach cancer, liver cancer, gallbladder cancer, biliary tract cancer, pancreatic cancer, small intestine cancer, colon cancer, anal cancer, bladder cancer, kidney cancer, male genital tumor, penile cancer, prostate cancer, female genital tumor, cervical cancer, endometrial cancer, ovarian cancer, uterine sarcoma, vaginal cancer, female external genital cancer, female urethral cancer or skin cancer.

In any one aspect of the present invention the compound may exhibit antitumor or anticancer activity by at least one action selected from the group consisting of:
(i) inhibition of tumor or cancer cell-specific proliferation by the induction of replicative senescence (RS) and/or oncogene-induced senescence (OIS);
(ii) reversal of the tumor or cancer cell NAD/NADH ratio;
(iii) upregulation of tumor or cancer cell ROS; and
(iv) inducing tumor or cancer cell-specific apoptosis, but the present invention is not limited thereto.

The composition of the present invention may be a composition mixed with the compound of the present invention or a salt thereof itself, or a pharmaceutically or sitologically acceptable carrier.

The composition of the present invention may contain 0.0001 to 100 wt.% of the compound of the present invention or a salt thereof, based on the total weight of the composition.

The composition of the present invention may be administered orally or parenterally during clinical administration, and when administered parenterally, it may be administered by intraperitoneal injection, intrarectal injection, subcutaneous injection, intravenous injection, intramuscular injection, intrauterine intrathecal injection, intracerebrovascular injection or intrathoracic injection, and it may be used in the form of general pharmaceutical formulations.

The composition of the present invention may be used alone or in combination with methods using surgery, radiation therapy, hormone therapy, chemotherapy and biological response modifiers.

The daily administration dose of the composition of the present invention may be about 0.0001 to 100 mg, or 0.001 to 10 mg per 1 kg of body weight based on the compound of the present invention or a salt thereof contained in the composition, and although it may be administered once or several times a day, the range will vary depending on the subject's weight, age, gender, health status, diet, administration time, administration method, excretion rate and the severity of disease.

For clinical administration, it may be formulated in various forms of oral or parenteral formulations, and in this case, diluents or excipients such as commonly used fillers, extenders, binders, wetting agents, disintegrants and surfactants may be used.

The pharmaceutical composition of the present invention may contain one or more active ingredients exhibiting the same or similar function in addition to the compound of the present invention or a pharmaceutically acceptable salt thereof.

The quasi-drug composition of the present invention refers to products with a milder action than pharmaceuticals among products used for the purpose of diagnosing, treating, improving, alleviating, treating or preventing diseases of humans or animals, and for example, according to the Pharmaceutical Affairs Act, quasi-drugs exclude products used for pharmaceutical purposes, and they include products used for the treatment or prevention of human or animal diseases, products with minor or no direct action on the human body and the like.

The quasi-drug composition of the present invention may be a transdermal dosage form such as a lotion, ointment, gel, cream, patch or spray. In each dosage form, the quasi-drug composition may be formulated by selecting other components arbitrarily according to the formulation or purpose of use of other quasi-drugs. The mixing amount of the active ingredient may be appropriately determined depending on the purpose of use (suppression or alleviation). For example, it may contain conventional adjuvants such as thickeners, stabilizers, solubilizers, vitamins, pigments and fragrances, carriers and the like. Further, in the quasi-drug composition of each dosage form, components other than the above essential components may be appropriately selected and formulated by those skilled in the art without difficulty depending on the dosage form or purpose of use.

The food composition of the present invention may be a composition mixed with the compound of the present invention or a pharmaceutically acceptable salt thereof itself, or a sitologically acceptable carrier. In this case, the content of the compound of the present invention or a pharmaceutically acceptable salt thereof may be appropriately adjusted according to the conventional method based on the content and administration dose in the pharmaceutical composition.

The most preferred aspect of the food composition of the present invention may be a health functional food composition, but the present invention is not limited thereto, and in another aspect, general food forms such as processed meat products, fish products, tofu, jelly, porridge, noodles such as ramen or noodles, seasonings such as soy sauce, soybean paste, gochujang, mixed soy sauce and the like, sauces, snacks, dairy products such as fermented milk or cheese, pickled foods such as kimchi or pickles, fruits, vegetables, soymilk, fermented beverages and the like are also possible. In another aspect, the food composition of the present invention may be a food additive.

Meanwhile, for the sitologically acceptable carrier, the pharmaceutically acceptable carrier may also be used.

### Example

Hereinafter, the present invention will be described in more detail through examples. These examples are only for illustrating the present invention, and it will be apparent to those of ordinary skill in the art that the scope of the present invention is not to be construed as being limited by these examples.

### Example 1. Anticancer effect on A549 cell line

### 1-1. Cell culture and reagents

The A549 cell line was purchased from the Korea Cell Line Bank and cultured in RPMI1640 (10% FBS + 1% P/S). Chemically synthesized ONG41008 (Chemical Formula 2) and ONG21001 (Eupatilin) were obtained from Syngene International Ltd. (Bangalore, India), dissolved in DMSO at a stock concentration of 50 mM, and stored in aliquots at -20°C. DMSO according to the concentration was used as a control. Hispidulin was purchased from Sigma (SML0582), Jaceosidin was purchased from Selleckchem (S0931), and Apigenin was purchased from Sigma (10798).

ONG41008 used as an example in the present invention is the compound of Chemical Formula 2 below.

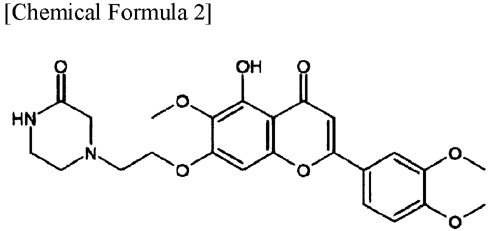

### 1-2. Cell senescence inducting effect of A549 cells by the compound of the present invention

A549 cells, which are a non-small cell lung cancer cell line, were inoculated in the medium and cultured for 24 hours, and the up- or down-regulated genes were analyzed by checking changes in the gene expression level with or without treatment with the compound of Chemical Formula 2 (ONG41008) of the present invention (20 µM).

To this end, cultured cells were washed twice with cold PBS and harvested by using TaKaRa MiniBEST Universal RNA Extraction Kit (Takara, Japan). RNA was purified by using the same kit according to the manufacturer's protocol. RNA was reverse transcribed by using a cDNA synthesis kit (PCRBio Systems, London, UK). The synthesized cDNA was amplified with StepOne Plus (Applied Biosystems, Life Technologies) and 2x qPCRBio Probe Mix Hi-ROX (PCRBio). Comparisons between mRNA levels were performed by using the ΔΔCt method, and GAPDH was used as an internal control.

Processed reads were mapped to the *Homo sapiens* reference genome by using Tophat and Cufflink with basic parameters. Differential analysis was performed by using Cuffdiff using the default parameters. In addition, Cuffdiff's FPKM values were normalized and quantified by using R TCC (comparison of the number of package tags) to determine statistical significance (e.g., P value) and differential expression (e.g., fold change). Gene expression values were plotted as Volcano plots by using fold change values using an in-house developed R script.

As a result, it was confirmed that the expression pattern of genes associated with cell senescence was remarkably changed according to the presence or absence of treatment with the compound of the present invention (data not illustrated).

By focusing on the fact that the compound according to the present invention is a chromone scaffold-containing derivative, immunocytochemistry was performed to confirm whether the effect of inducing cell senescence is also exhibited in other chromone scaffold-containing derivatives.

In the A549 cell line, 20 µM of each of the compound of Chemical Formula 2 (ONG41008) of the present invention, eupatylin (ONG21001) or other chromone scaffold-containing derivatives such as hispidulin, jaceosidin and apigenin was treated to the A549 cell line, and after 72 hours of treatment, cells were fixed by using 4% paraformaldehyde, permeabilized with 0.4% TritonX100 and blocked with 1% BSA. The cells were incubated with Rhodamine Phalloidin (Thermo Fisher, Massachusetts, US) for 4 hours at room temperature. After washing, the cells were incubated with Alexa Fluor 488 (Abeam, Cambridge, UK)-conjugated secondary antibody. Images were analyzed by using EVOS M7000 (Invitrogen, CA, USA). Nuclei were counterstained with DAPI.

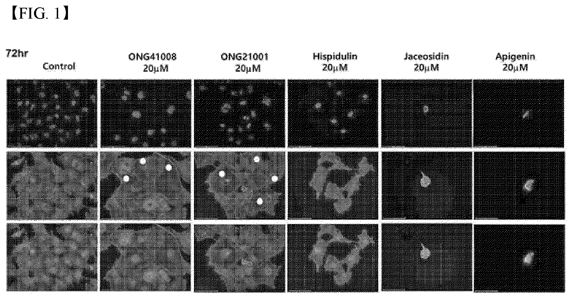

As a result, as shown in FIG. 1, cell flatness, which is a morphological change of cell senescence, was specifically observed in cells treated with the compound of Chemical Formula 2 (ONG41008) of the present invention and eupatylin (ONG21001). That is, a squamous cell morphology was observed in A549 cells treated with the compound of Chemical Formula 2 (ONG41008) of the present invention and eupatylin (ONG21001), but in the experimental groups treated with other chromone scaffold-containing derivatives, the morphological characteristics of cell senescence were not observed, and thus, it was found that cell senescence is a compound-specific effect according to the present invention among various chromone scaffold-containing derivatives.

### 1-3. Replication senescence of A549 cells by the compound of the present invention

Several tumor suppressor proteins are known to be involved in the arrest of cell cycle progression. TP53 is phosphorylated and inhibits the cell cycle mainly by binding to p21 or p16. Immunocytochemistry was performed to confirm that the compound treatment according to the present invention induces replication senescence centered on TP53-p21-p16 in A549 cells.

A549 cell line was treated with 1, 5, 10 and 20 µM of the compound of the present invention (ONG41008), respectively, and after 24 or 72 hours, the cells were fixed by using 4% paraformaldehyde, permeabilized with 0.4% TritonX100 and blocked with 1% BSA. The cells were incubated with Anti-p53 (Cell Signaling Technology, Beverly, MA), Anti-p21 (Abcam, Cambridge, UK) and Anti-p16-INK4A (Proteintech, IL, USA) at room temperature for 4 hours. After washing, the cells were incubated with Alexa Fluor 488 (Abcam, Cambridge, UK)-conjugated secondary antibody. Images were analyzed by using EVOS M7000 (Invitrogen, CA, USA). Nuclei were counterstained with DAPI.

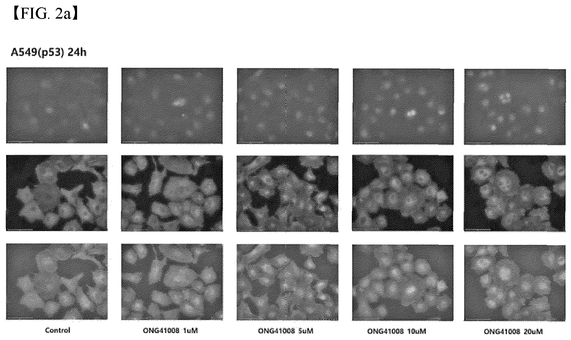

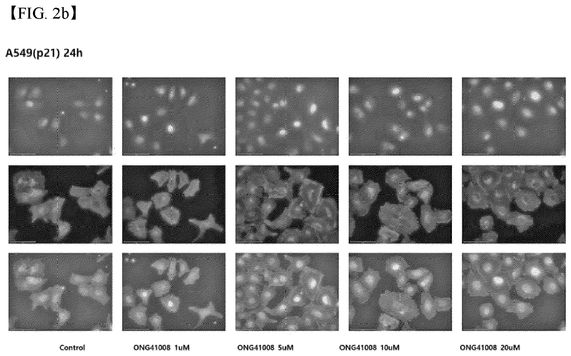

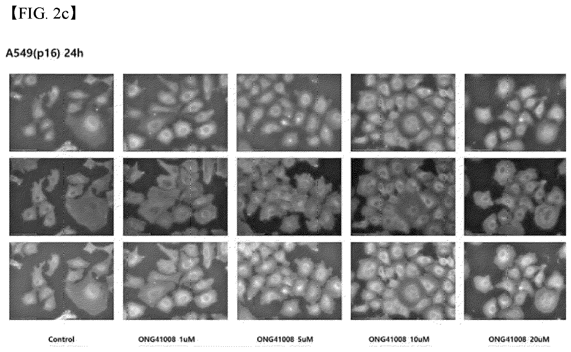

As a result, as shown in FIGS. 2a to 2c, TP53 protein expression level was not changed upon treatment with 1 µM, but nuclear localization was observed, and p21 was nuclear localized with upregulation. p16 was up-regulated in proportion to the concentration of the compound of Chemical Formula 2 (ONG41008) of the present invention and was simultaneously located in the nucleus and the nucleus periphery.

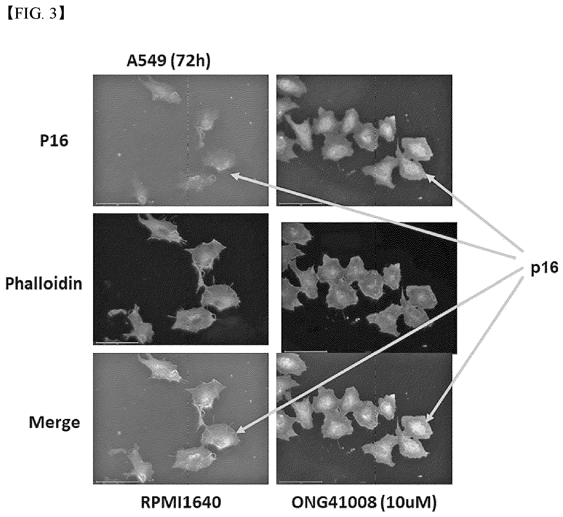

It was confirmed that the cell morphology became more uniform at 72 hours of compound treatment than at 24 hours of compound treatment, reflecting the removal of multinucleated A549 cells. The maximum translocation of p16 to the nucleus appears to be completed at 72 hours, and thus, it is predicted that A549 cells saturated with p16 will apoptosis (FIG. 3).

Since the phosphorylation of TP53 is known to be essential for regulating cell cycle arrest, Western blotting was performed by using a phospho-specific TP53 antibody.

To this end, A549 cells were seeded in a 100 mm cell culture dish at 1 × 10⁶ cells/well, cultured overnight and then treated with various concentrations of the compound of Chemical Formula 2 (ONG41008) of the present invention. After 24 hours, the cell lysates were clarified by centrifugation at 14,000 × g for 10 minutes, and the supernatant was collected. Protein concentration was quantified by Bradford assay (Thermo Fisher, Massachusetts, USA). Afterwards, 25 µg of cellular protein was loaded onto a 10% SDS-PAGE gel and transferred to a nitrocellulose membrane. After blocking with 5% BSA, the membrane was incubated overnight at 4°C with Anti-p53 (Cell Signaling Technology, Beverly, MA), Anti-phosphor-p53 (Cell Signaling Technology, Beverly, MA), Anti-p21 (Abcam, Cambridge, UK), Anti-p16-INK4A (Proteintech, IL, USA) and Anti-GAPDH (Abcam, Cambridge, UK). After thorough washing, the membrane was incubated with HRP-conjugated secondary antibody. Protein bands were visualized by using ECL reagent (Abfrontier, Korea) and Uvitec HD9 (UVITEC, UK).

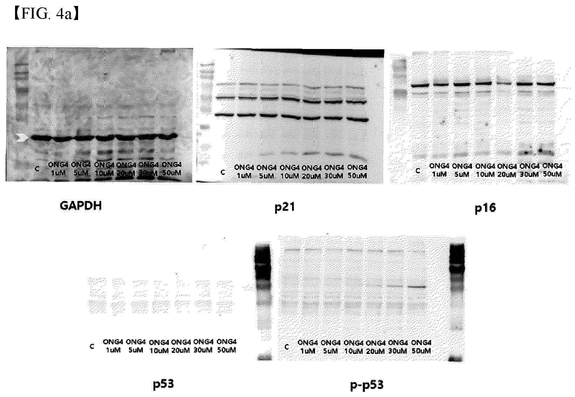

As a result, TP53 was phosphorylated in proportion to the increase in the concentration of the compound of Chemical Formula 2 (ONG41008) of the present invention (FIG. 4a), and the total amount of TP53 was maintained to be the same. In addition, both of p21 and p16 were induced in a concentration-dependent manner, and it was consistent with the results of FIGS. 2a to 2c.

Meanwhile, in order to further proliferate A549 cells, TGFβ was treated to stimulate A549 cells, and in this case, cell changes were observed with or without treatment with the compound of Chemical Formula 2 (ONG41008) (10 µM) of the present invention.

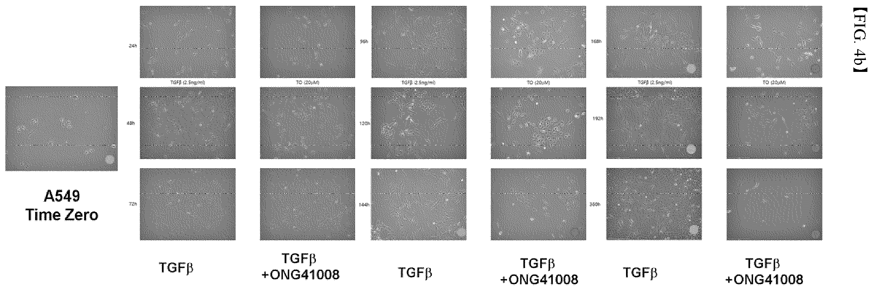

As a result, it was observed that a significant portion of the A549 cells treated with the compound Chemical Formula 2 (ONG41008) of the present invention started to die from the 6^{th} day, and complete apoptosis was induced on the 15^{th} day, whereas the control A549 maintained an energetic state. Blue and red circles represent control A549 cells and ONG41008 compound-treated A549 cells, respectively. All cells exposed to 10 µM of the compound of Chemical Formula 2 (ONG41008) of the present invention appeared to receive an apoptosis signal for 72 hours (FIG. 4b).

### 1-4. Cell senescence-mediated apoptosis of A549 cells by the compound of the present invention

A549 cells were rapidly converted to replicative senescence by the compound of the present invention, and since cell apoptosis was predicted to be induced, cell apoptosis was observed following treatment with the compound of the present invention. One of the characteristics of apoptosis is multinucleation (MNC), and particularly, the multinucleation of cancer cells is known to be caused by oncogene-induced senescence (OIS).

Cell senescence was established by treating A549 cells with 10 µM of the compound of Chemical Formula 2 (ONG41008) of the present invention for 24 hours, 48 hours or 72 hours, and immunocytochemistry was performed to observe multinucleation along with morphological changes. ZEB1, which is an epithelial-mesenchymal transition (EMT) positive regulator, was used as an expression control.

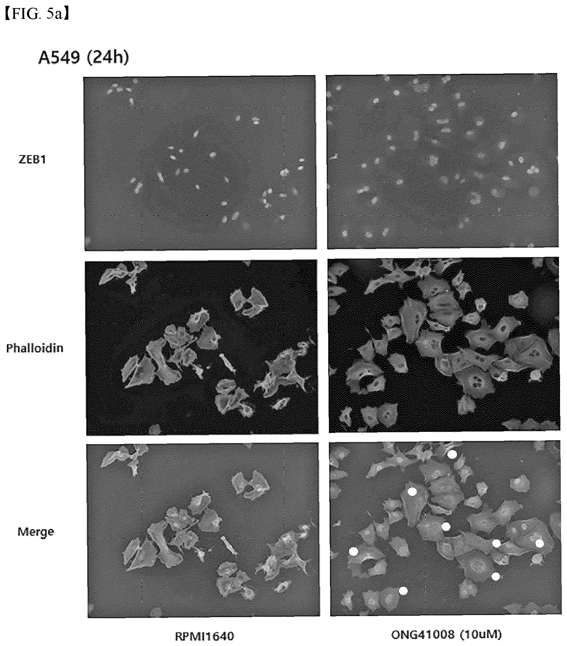

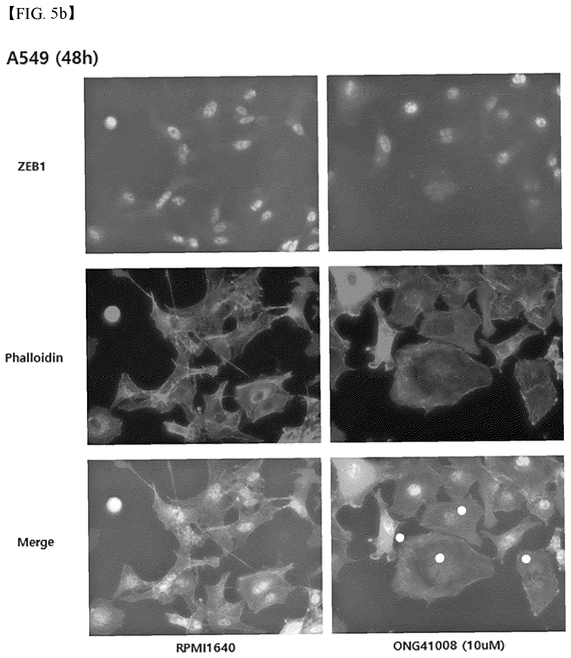

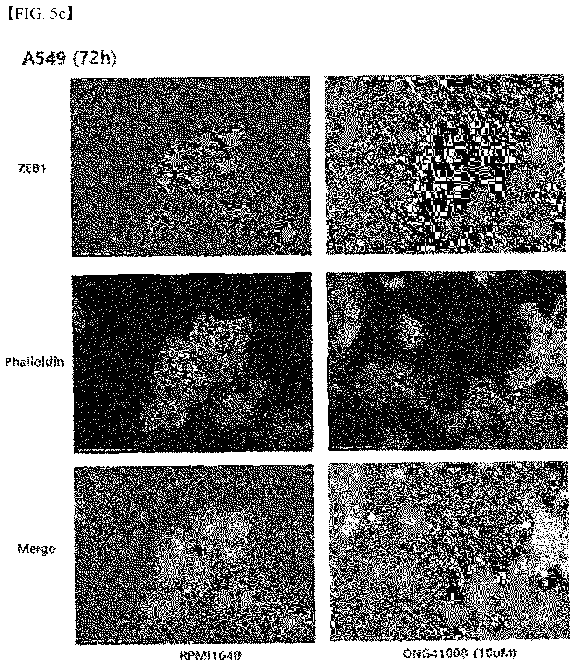

As a result, cell flattening was clearly confirmed in A549 at 24 hours of treatment with the compound of Chemical Formula 2 (ONG41008) of the present invention, and multinucleation was also observed, and the greatest number of multinucleation was observed after 48 hours of treatment, and rather at 72 hours of treatment, the number thereof decreased, which is expected as a result of apoptosis (FIGS. 5a to 5c).

### 1-5. Aging by induction of oncogenes of A549 cells by the compound of the present invention

The mode of action (MOA) of the compound of the present invention to eliminate cancer cells is expected to be due to oncogene induction-induced senescence. Meanwhile, in general, anticancer drugs target cell cycle regulation, DNA replication or chromatin remodeling, and in A549 cells treated with the compound of the present invention, changes in the expression level of HIST1H4K (H4 Clustered Histone 12) involved in DNA replication and DNA repair were observed (data not shown). Accordingly, the effects of suberoylanilide hydroxamic acid (SAHA), which is a reversible pan-histone deacetylase (HDAC) inhibitor, and the compound of the present invention on cell cycle regulation were compared.

To this end, A549 cells were aliquoted in 96-well plates for 24 hours, and then exposed to different concentrations of the compound of Chemical Formula 2 (ONG41008) of the present invention or SAHA. Afterwards, cells were co-incubated with TMRE (Abcam, ab113852) at 37°C in the dark for 30 minutes, and the mitochondrial membrane potential was examined according to the manufacturer's protocol. 20 µM FCCP treated cells were used as a positive control.

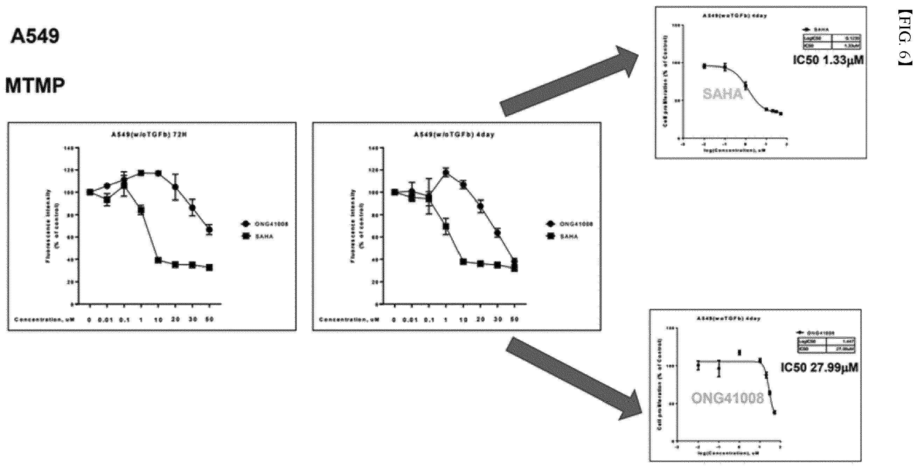

As a result, it was confirmed by mitochondrial membrane potential analysis that the cell viability rapidly decreased at 24 hours after SAHA treatment, and the substantial survival of the cells was maximally inhibited at 72 hours. On the other hand, the inhibitory effect of the compound of Chemical Formula 2 (ONG41008) of the present invention started at 48 hours and continued to increase until day 4 until all cells were removed. This was in a similar context to the data by immunocytochemistry, which confirmed that aging due to oncogene induction started at 48 hours of treatment with the compound of Chemical Formula 2 (ONG41008) of the present invention and continued until 72 hours (FIGS. 5a to 5c). The IC₅₀ of SAHA and ONG41008 compounds showed about a 20-fold difference (FIG. 6).

In summary, SAHA induces rapid apoptosis due to the broad HDAC inhibitory effect thereof on the chromatin remodeling process, but also induces various undesirable effects in this process, whereas it is interpreted that the compound of the present invention induces apoptosis through oncogene-induced senescence, and due to this difference, it is determined that the apoptosis-inducing effect and duration of the present invention are excellent.

### 1-6. G2/M cell cycle arrest and NAD/NADH ratio change in A549 cells by the compound of the present invention

The association between the compound of the present invention and TP53, p16 and p21 means that the compound of the present invention plays an important role in cell cycle control. In order to confirm how the compound of the present invention acts on the cell cycle, A549 cells were treated with the compound of the present invention, Western blot was performed at 24 hours, and PI staining was performed at 24 and 48 hours, respectively, to perform cell cycle analysis.

For Western blotting, A549 cells were seeded in a 100 mm cell culture dish at 1 × 10⁶ cells/well and cultured overnight, followed by treatment of 50 µM of the compound of Chemical Formula 2 (ONG41008) of the present invention or 50 µM of Fisetin (Glantham, GL7384-100MG). Fisetin is a cell senescence inducer and induces the arrest of the G0/G1 phase. After 24 hours, the cell lysate was clarified by centrifugation at 14,000 ×g for 10 min, and the supernatant was collected. Protein concentration was quantified by Bradford assay (Thermo Fisher, Massachusetts, USA). Thereafter, 25 µg of cellular protein was loaded onto a 10% SDS-PAGE gel and transferred to a nitrocellulose membrane. After blocking with 5% BSA, membranes were incubated overnight at 4°C with Anti-CDK2 (Cell Signaling Technology, MA), Anti-CDK4 (Cell Signaling Technology, MA) and Anti-GAPDH (Abcam, Cambridge, UK) antibodies. After thorough washing, the membranes were incubated with HRP-conjugated secondary antibody. Protein bands were visualized by using ECL reagent (Abfrontier, Korea) and Uvitec HD9 (UVITEC, UK).

For PI staining, A549 cells were aliquoted in a 100 mm cell culture dish at 2 × 10⁵ cells/mL, harvested 24 hours later and washed with PBS, and the compound of Chemical Formula 2 (ONG41008) of the present invention was added at 10 µM, 20 µM, 50 µM or control medium was added, respectively. After 24 hours, the cells were harvested and fixed in cooled 70% ethanol, and then stained with propidium iodide containing 200ug/mL RNase (Abcam, 139418) in the dark at 37°C for 30 minutes. Each phase of the cell cycle was identified by using Accuri C6 Plus (BD Bioscience).

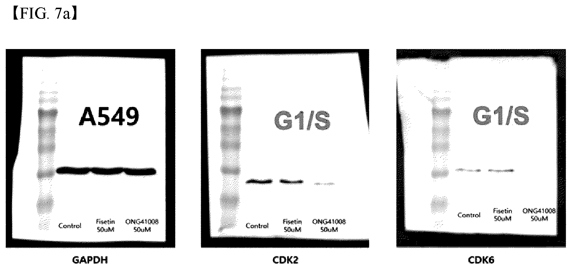

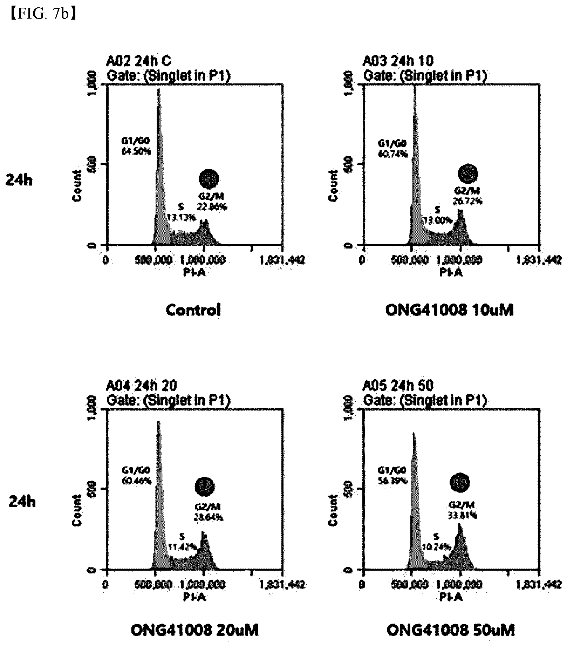

As a result, it was confirmed that the compound of Chemical Formula 2 (ONG41008) of the present invention inhibited the expressions of CDK2 and CDK6 at 50 µM to induce arrest of the G1-S-G2 phase (FIGS. 7a and 7b). Therefore, it was found that the compound of the present invention causes mitotic collapse by oncogene-induced senescence (OIS), and consequently induces apoptosis.

Metabolic regulation has been regarded as a key driver for controlling cell cycle regulation or apoptosis. It is well known that the NAD/NADH ratio plays a central role in regulating energy metabolism, including glycolysis and the TCA cycle, and has a significant impact on mitochondrial functions such as onset or aging. Accordingly, it was attempted to determine whether the compound of the present invention affects the NAD/NADH ratio in A549 cells.

To this end, total NAD was extracted and quantified from A549 cell lysates by using the NAD+/NADH Colorimetric Assay Kit (Abcam, ab65348) according to the manufacturer's protocol. 1 × 10⁶ cells were lysed in 400 µL NAD/NADH extraction buffer, filtered through a 10 kD spin column (ab93349) and measured in pure water or 1/5 dilution. Briefly, the amount of total NAD was calculated by dividing the standard curve (pmol) by the sample volume (µL) added to the reaction wells and multiplying by the dilution factor.

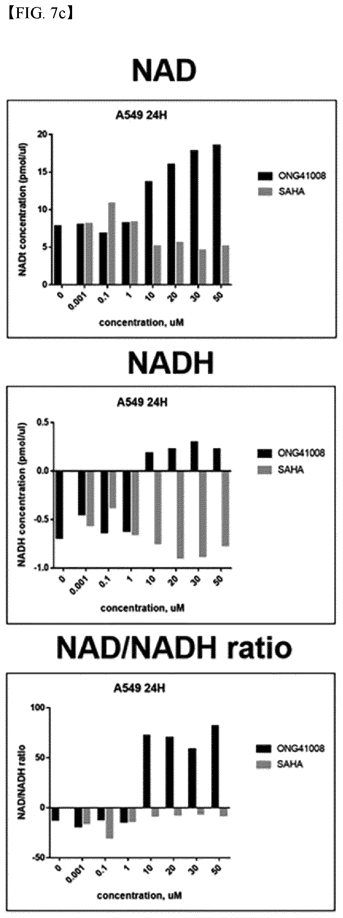

As a result, it was confirmed that when A549 cells were stimulated with the compound of Chemical Formula 2 (ONG41008) of the present invention, NAD biosynthesis was increased, and the NAD/NADH ratio was significantly increased (FIG. 7c). On the other hand, SAHA did not induce this effect at all. It can be seen that the NAD/NADH ratio of A549 cells itself shows a negative value, indicating that A549 cells can exclusively utilize aerobic glycolysis. Since the compound of the present invention can enhance the intracellular concentration of NAD, the rate limiting enzyme NAMPT (nicotinamide phosphoribosyltransferase) in this pathway may be the main target of the compound of the present invention.

The compound of the present invention can normalize the NAD/NADH ratio in the range of 60 to 100, and it was confirmed whether the normalized NAD/NADH ratio affects ROS production in cancer cells treated with the compound according to the present invention.

To this end, 1 × 10⁴ cells/mL of A549 cells were dispensed in 96-well plates, and after 24 hours, the cells were treated with 10 µM H2DCFDA (Invitrogen, CA) and incubated at 37°C in the dark for 1 hour. Thereafter, the compound of Chemical Formula 2 (ONG41008) of the present invention or TBHP (Abcam, UK), which is a ROS inducer as a positive control, was treated at various concentrations for 24 hours. Intracellular ROS were examined by fluorescence microscopy (Thermo, MA) and quantified by fluorescence spectroscopy (Perkin Elmer, MA).

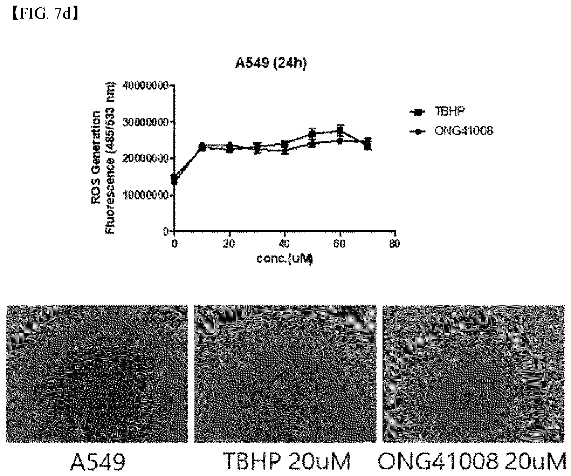

As a result, when the compound of Chemical Formula 2 (ONG41008) of the present invention was treated, it was found that ROS increased in a concentration-dependent manner (FIG. 7d), and this is interpreted as increasing the NAD/NADH ratio and affecting the ROS production of cancer cells to cause oncogene-induced senescence.

Meanwhile, as can be seen in the comparative examples, the compound of the present invention acts specifically for cancer cells by discriminating between cancer cells and normal cells, and this may be because the chromone scaffold of the compound of the present invention may recognize some of the components constituting an intracellular microenvironment including NAD, NADH, pH or ATP as energy sensors.

### 1-7, Analysis of apoptosis kinetics according to the treatment of the compound of the present invention

In order to observe the apoptosis of A549 cells over time after treatment with the compound of the present invention, TUNEL analysis and 7AAD analysis were performed.

The TUNEL analysis was performed by using the TUNEL assay kit (Abcam, ab66110) according to the manufacturer's instructions. A549 cells were seeded in a 100 mm cell culture dish at 2 × 10⁵ cells/mL and harvested 24 hours later, and after washing with PBS, 10 µM, 20 µM and 50 µM of the compound of Chemical Formula 2 (ONG41008) of the present invention or control medium was added thereto, respectively. After 24 hours, the cells were harvested and fixed in cold 70% ethanol, and 50 µL of a DNA labeling solution (10 µL of TdT reaction buffer, 0.75 µL of TdT Enzyme, 8 µL of Br-dUTP, 32.5 µL of ddH₂O) was added thereto. After 1-hour incubation at 37°C, the cells were washed twice with 300 µL of rinse buffer, and an antibody solution (5 µL of Anti-BrdU-Red antibody, 95 µL rinse buffer) was prepared and added to the washed cells, and the cells were incubated in the dark at room temperature for 30 minutes. 300 µL of 7-AAD/RNase solution was added at last. Each sample was analyzed by using Accuri C6 Plus (BD Bioscience).

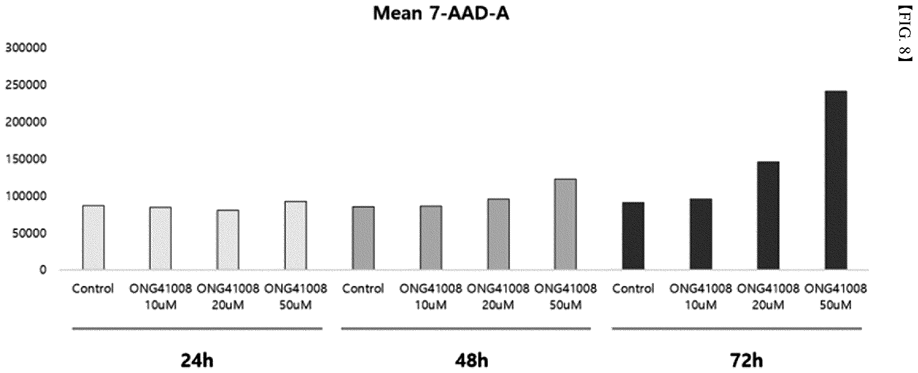

As a result, apoptosis was observed at 48 hours of treatment with the compound of Chemical Formula 2 (ONG41008) of the present invention, and apoptosis was clearly observed in a compound concentration-dependent manner at 72 hours of treatment with the compound of Chemical Formula 2 (ONG41008) of the present invention (FIG. 8).

### Example 2. Anticancer effect on H358 and H1299 cells

### 2-1, Cell culture and reagents

Non-small cell lung cancer primary cells, H358 and H1299, were both purchased from the Korea Cell Line Bank and cultured in RPMI1640 (10% FBS + 1% P/S). Chemically synthesized ONG41008 was obtained from Syngene International Ltd. (Bangalore, India), dissolved in DMSO at a stock concentration of 50 mM, and stored in aliquots at -20°C.

### 2-2. Inhibition of proliferation of H358 cells by the compound of the present invention

The effect of inhibiting the proliferation of H358 cells by the compound of the present invention was confirmed by CCK-8 analysis and compared with the effect of paclitaxel, which is an anti-cancer therapeutic agent approved for use in the treatment of ovarian cancer, breast cancer, lung cancer or gastric cancer.

H358 cells were seeded in 96-well plates at a concentration of 3 × 10⁴ cells/well for 24 hours. The compound of Chemical Formula 2 (ONG41008) of the present invention or paclitaxel (Abcam, ab120143) was treated at 0.1, 1, 10, 20, 30 and 50 µM, respectively, and cell viability after 72 hours was measured by using cell counting kit-8 (Dojindo, CK04) according to the manufacturer's protocol. In addition, after 72 hours of treatment with the compound of Chemical Formula 2 (ONG41008) of the present invention, H358 cells were observed with an optical microscope (EVOS M7000).

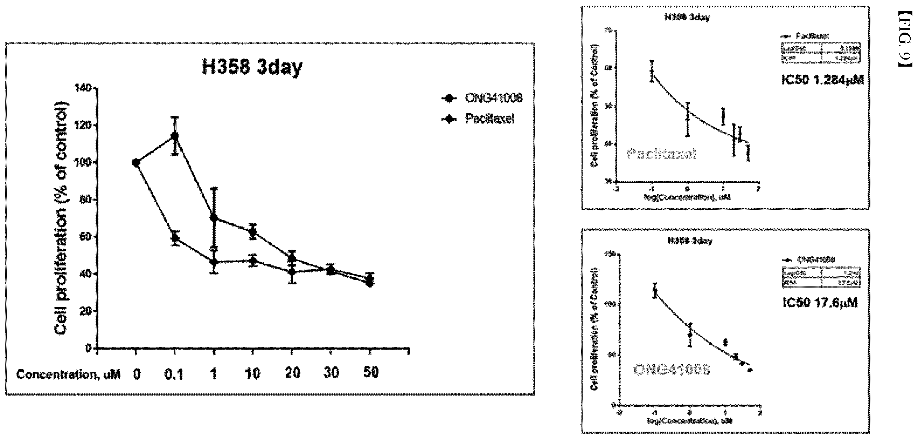

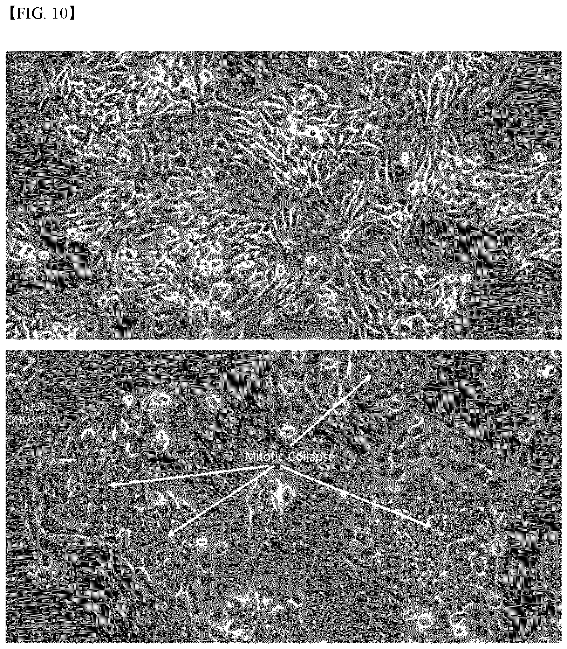

As a result of the experiment, both of the compound of the present invention (ONG41008) and paclitaxel inhibited the proliferation of non-small cell lung cancer cells to a similar level when treated with 30 µM or more, except that the IC₅₀ of paclitaxel and ONG41008 showed a 14-fold difference (FIG. 9). Meanwhile, the mitotic disruption of H358 cells was observed after 72 hours of treatment with 10 µM of the compound of Chemical Formula 2 (ONG41008) of the present invention (FIG. 10).

### 2-3. Multinucleation of H1299 cells by the compound of the present invention

The compound of Chemical Formula 2 (ONG41008) of the present invention was treated by concentration, and immunocytochemistry was performed in the same manner as in Example 1 to observe cell changes.

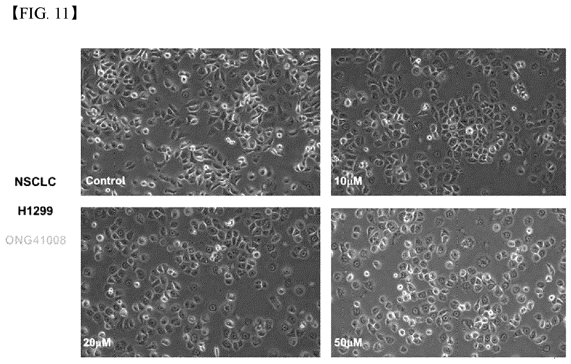

As a result of the experiment, as shown in FIG. 11, it was confirmed that multinucleation, which is an indicator of senescence due to oncogene induction, was observed according to the treatment with the compound of Chemical Formula 2 (ONG41008) of the present invention.

### Example 3. Anticancer effect on PANC1 cells

### 3-1. Cell culture and reagents

PANC1 cells, which are a human pancreatic cancer cell line, were purchased from the Korea Cell Line Bank and cultured in RPMI1640 (10% FBS + 1% P/S).

Chemically synthesized ONG41008 was obtained from Syngene International Ltd. (Bangalore, India), dissolved in DMSO at a stock concentration of 50 mM and stored in aliquots at -20°C.

### 3-2. Inhibition of proliferation of PANC1 cells by the compound of the present invention

The effect of inhibiting the proliferation of PANC1 cells by the compound of Chemical Formula 2 (ONG41008) of the present invention was confirmed by CCK-8 analysis and compared with the effect of paclitaxel.

The experimental method was carried out in the same manner as described in Example 2 except for the cells used.

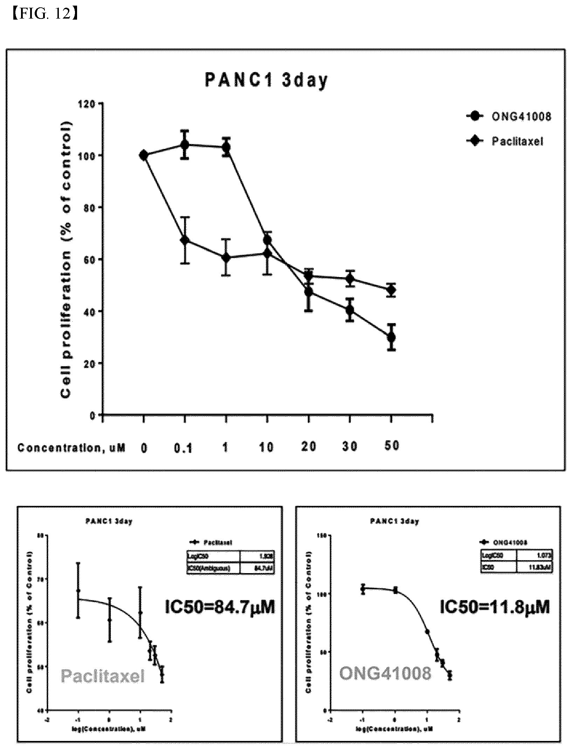

As a result of the experiment, the compound of Chemical Formula 2 (ONG41008) of the present invention exhibited a more excellent inhibitory effect on the proliferation of pancreatic cancer cells at a concentration of 10 µM or more compared to paclitaxel, and it was confirmed that the IC₅₀ of the compound of Chemical Formula 2 (ONG41008) of the present invention for PANC1 was 11.8 µM, and the IC₅₀ of paclitaxel for PANC1 was 84.7 µM (FIG. 12).

### 3-3. Multinucleation of PANC1 cells by the compound of the present invention

The compound of Chemical Formula 2 (ONG41008) of the present invention was treated by concentration, and immunocytochemistry was performed in the same manner as in Example 1 to observe cell changes.

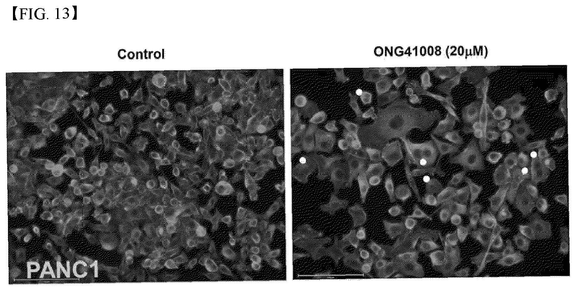

As a result, as shown in FIG. 13, multinucleation, which is an indicator of senescence due to oncogene induction, was observed according to the treatment with the compound of Chemical Formula 2 (ONG41008) of the present invention.

### Example 4. Anticancer effect on MCF7 cells

### 4-1, Cell culture and reagents

MCF7 cells, which are a human breast cancer cell line, were purchased from the Korea Cell Line Bank and cultured in RPMI1640 (10% FBS + 1% P/S).

Chemically synthesized ONG41008 was obtained from Syngene International Ltd. (Bangalore, India), dissolved in DMSO at a stock concentration of 50 mM and stored in aliquots at -20°C.

### 4-2. Inhibition of proliferation of MCF7 cells by the compound of the present invention

The effect of inhibiting the proliferation of MCF7 cells by the compound of Chemical Formula 2 (ONG41008) of the present invention was confirmed by CCK-8 analysis and compared with the effect of SAHA.

The experimental method was carried out in the same manner as described in Example 2 except for the cells used and the positive control reagent.

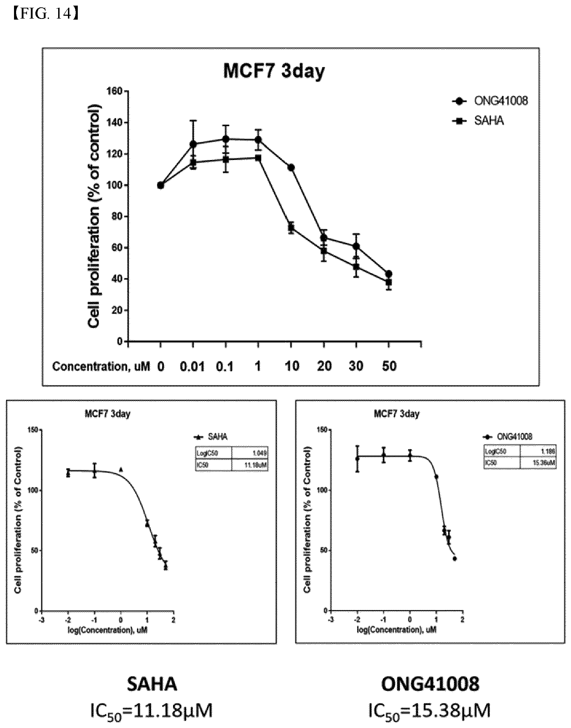

As a result of the experiment, the compound of Chemical Formula 2 (ONG41008) of the present invention exhibited a cell proliferation inhibitory effect that was very similar to that of SAHA, and it was confirmed that the IC₅₀ of the compound of the present invention (ONG41008) for MCF7 was 15.38 µM, and the IC₅₀ of SAHA for MCF7 was 11.18 µM (FIG. 14).

### 4-3. Multinucleation of MCF7 cells by the compound of the present invention

The compound of Chemical Formula 2 (ONG41008) of the present invention was treated by concentration, and immunocytochemistry was performed in the same manner as in Example 1 to observe cell changes.

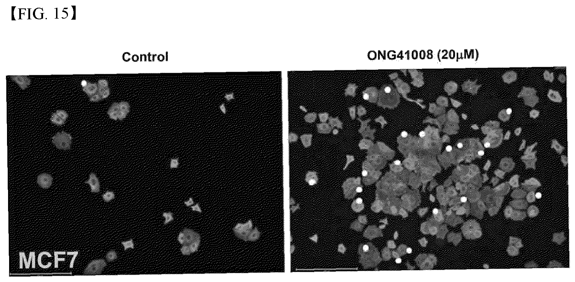

As a result of the experiment, as shown in FIG. 15, multinucleation, which is an indicator of senescence due to oncogene induction, was observed according to the treatment with the compound of Chemical Formula 2 (ONG41008) of the present invention.

### Example 5. Anticancer effect on PC3 cells

### 5-1, Cell culture and reagents

PC3 cells, which are a human prostate cancer cell line, were purchased from the Korea Cell Line Bank and cultured in RPMI1640 (10% FBS + 1% P/S).

Chemically synthesized ONG41008 was obtained from Syngene International Ltd. (Bangalore, India), dissolved in DMSO at a stock concentration of 50 mM and stored in aliquots at -20°C.

### 5-2. Inhibition of proliferation of PC3 cells by the compound of the present invention

The effect of inhibiting the proliferation of PC3 cells by the compound of Chemical Formula 2 (ONG41008) of the present invention was confirmed by CCK-8 analysis and compared with the effect of SAHA.

The experimental method was carried out in the same manner as described in Example 2 except for the cells used and the positive control reagent.

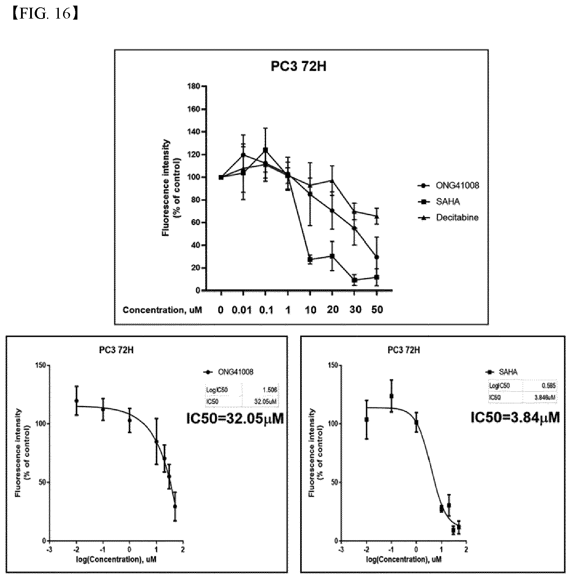

As a result of the experiment, the cell proliferation inhibitory effect of the compound of Chemical Formula 2 (ONG41008) of the present invention was confirmed, and specifically, it was confirmed that the IC₅₀ of the compound of Chemical Formula 2 (ONG41008) of the present invention for PC3 was 32.05 µM, and the IC₅₀ of SAHA for PC3 was 3.84 µM (FIG. 16). Although the IC₅₀ for PC3 was confirmed to be lower for SAHA, it was confirmed that the compound of Chemical Formula 2 (ONG41008) of the present invention induced apoptosis of PC3 cells at a level similar to that of SAHA over time.

### 5-3. Induction of apoptosis of PC3 cell by the compound of the present invention

The effect of inducing apoptosis of PC3 cells by the compound of Chemical Formula 2 (ONG41008) of the present invention was confirmed by Caspase-3/7 activity, and was compared with the effect by SAHA.

Caspase-3/7 activity was measured by using a Caspase-3/7 assay kit (Promega, G8091). Cells that were treated at various concentrations of the compound of Chemical Formula 2 (ONG41008) of the present invention or SAHA were harvested and lysed on ice. Thereafter, protein was measured with BCA (Thermo, 23227), and the protein was adjusted to 50 µg per 50 µL of cell lysis buffer. Afterwards, Caspase-3/7 activity was analyzed according to the manufacturer's protocol.

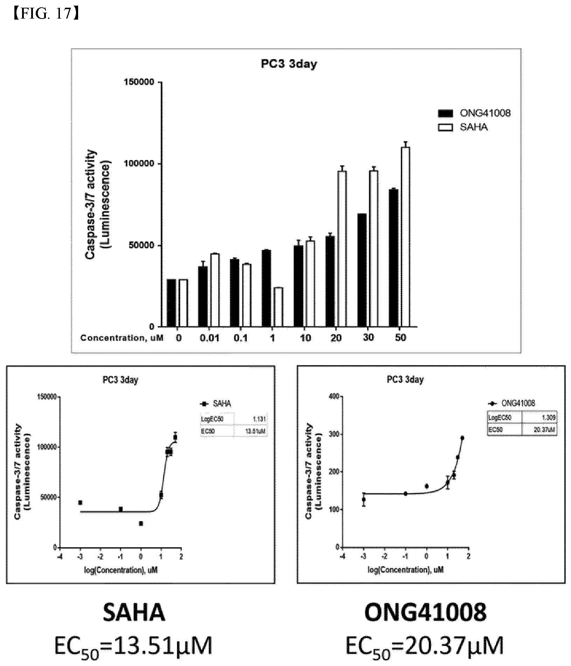

As a result of the experiment, as shown in FIG. 17, it was confirmed that both of the compound of Chemical Formula 2 (ONG41008) of the present invention and SAHA induced PC3 apoptosis in a concentration-dependent manner. In this case, it was confirmed that the EC₅₀ of the compound of Chemical Formula 2 (ONG41008) of the present invention was 20.37 µM, and the EC₅₀ of SAHA was 13.51 µM.

### 5-4. Multinucleation of PC3 cells by the compound of the present invention

The compound of Chemical Formula 2 (ONG41008) of the present invention was treated by concentration, and immunocytochemistry was performed in the same manner as in Example 1 to observe cell changes.

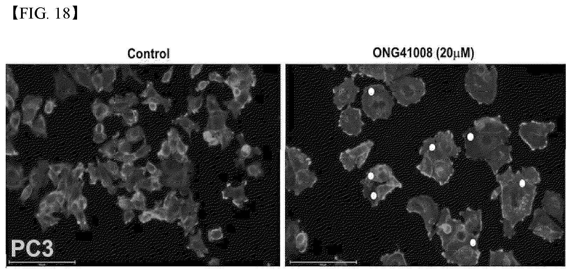

As a result, as shown in FIG. 18, multinucleation, which is an indicator of senescence due to oncogenic induction, was observed according to the treatment with the compound of Chemical Formula 2 (ONG41008) of the present invention.

### Comparative Example: Effect of the compound of the present invention on normal cells

### 1-1. Cell culture and reagents

Normal human lung fibroblasts (NHLF) were purchased from Lonza (CC-2512) and cultured in Fibroblast Growth medium (0.1% insulin, 0.1% hFGF-B, 0.1% GA-1000, 2% FBS).

Chemically synthesized ONG41008 was obtained from Syngene International Ltd. (Bangalore, India), dissolved in DMSO at a stock concentration of 50 mM and stored in aliquots at -20°C.

### 1-2. Confirmation of inhibition of cell proliferation by the compound of the present invention

It was confirmed by CCK-8 analysis whether the cell proliferation of NHLF cells, which are normal cells, was inhibited by the compound of Chemical Formula 2 (ONG41008) of the present invention. In this case, the effects on normal cells were compared with SAHA and nintedanib, which is a commercialized anticancer drug.

The experimental method was carried out in the same manner as described in Example 2 except for the cells used and the positive control reagent.

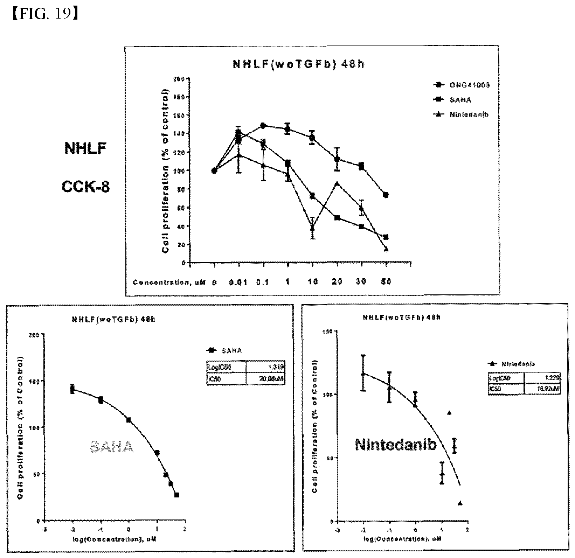

As a result of the experiment, as shown in FIG. 19, both of SAHA and nintedanib inhibited cell proliferation and exhibited tremendous toxicity to normal human lung fibroblastoma (NHLF), but the compound of Chemical Formula 2 (ONG41008) of the present invention did not inhibit cell proliferation

### 1-3. Confirmation of apoptosis by the compound of the present invention

In order to confirm whether the apoptosis of NHLF cells is induced by the compound of Chemical Formula 2 (ONG41008) of the present invention, (i) mitochondrial membrane potential analysis, (ii) Caspase-3 activity analysis and (iii) LDH cytotoxicity analysis were performed.
(i) Mitochondrial membrane potential analysis was performed in the same manner as in Example 1.
(ii) Caspase-3 activity assay was measured by using a Caspase-3 assay kit (Abcam, ab37401). Briefly, cells that were treated with the compound of Chemical Formula 2 (ONG41008) of the present invention or nintedanib at various concentrations were harvested and lysed on ice. Thereafter, protein was measured with BCA (Thermo, 23227), and the protein was adjusted to 50 µg per 50 µL of cell lysis buffer. Afterwards, Caspase-3 activity was analyzed according to the manufacturer's protocol.
(iii) LDH cytotoxicity assay detected LDH release by using an LDH assay kit (Abcam, ab56393). Briefly, cell culture plates that were treated at various concentrations of the compound of Chemical Formula 2 (ONG41008) of the present invention, nintedanib or SAHA were centrifuged at 480 × g for 10 minutes, and after the supernatant (10 µL/well) was extracted to another 96-well plate, 100 µL of LDG reaction mixture was added to each well and incubated for 30 minutes at room temperature. Absorbance values were measured at 450 nm in a microplate reader.

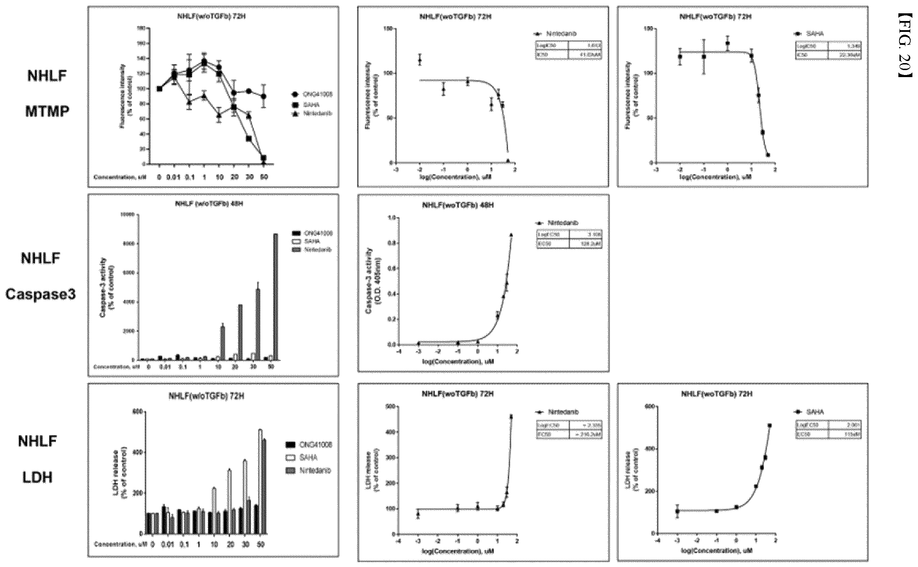

As a result of the experiment, as shown in FIG. 20, when measured with MTMP, Caspase3 and LDH, SAHA and nintedanib induced strong apoptosis even in normal cells ([SAHA] MTMP: IC 50 22.36 µM, LDH: EC 50 115.0 µM / [Nintedanib] MTMP: IC 50 41.02 µM, Caspase3: EC 50 128.2 µM, LDH: EC 50 218.2 µM), the compound of Chemical Formula 2 (ONG41008) of the present invention did not induce significant apoptosis in normal cells.

Meanwhile, even in the additional Caspase3/7 analysis, the compound of Chemical Formula 2 (ONG41008) of the present invention did not induce caspase3/7 activity unlike in the cancer cell line (PC3), and thus, it was confirmed once again that the compound of Chemical Formula 2 (ONG41008) of the present invention did not induce apoptosis in normal cells (data not shown).

The compound according to the present invention is a potent inducer of tumor cell or cancer cell-specific cell senescence (replicative senescence (RS) and oncogene-induced senescence (OIS)), and it liberates these cells from uncontrolled proliferative loops by way of cellular senescence, and at the same time induces cancer cell-specific apoptosis to exhibit excellent anti-tumor or anti-cancer properties.

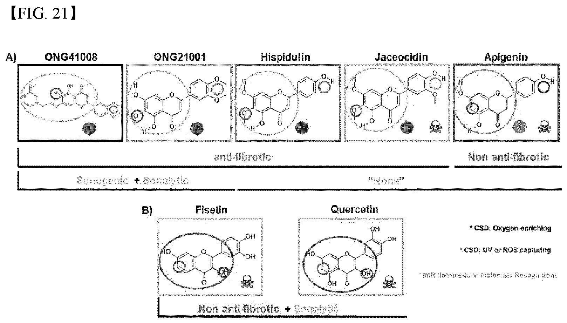

This property can be said to be a unique property that is exhibited only by the compound of the present invention among various chromone scaffold derivatives, and as other chromone scaffold derivatives, Histidulin and Jaceocidin having an oxygen at the C6 position exhibit anti-fibrotic effects, but they do not exert any effect of inducing cancer cell-specific cell senescence or eliminating senescent cells, Apigenin does not induce all of the effects of anti-fibrosis, cancer cell-specific cell senescence or senescent cells, and Fisetin and Quercetin induce apoptosis, but do not remove senescent cells or induce anti-fibrotic effects. Therefore, since the compound of the present invention can effectively remove (Senolytic) senescent cells with cancer cell-specific cell senescence (Senogenic), it is interpreted as exhibiting excellent anti-tumor or anti-cancer properties as confirmed in the present invention (refer to FIG. 21).

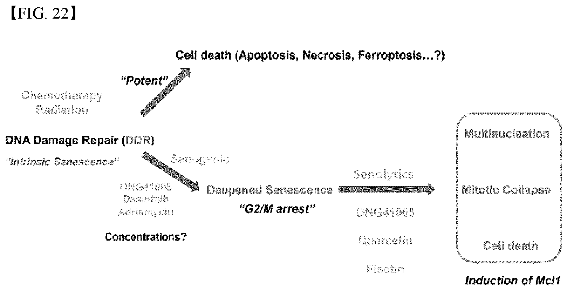

Recently, there have been reports of concurrent administration of Dasatinib and Quercetin or concurrent administration of Doxorubicin (Adriamycin^{®}) and Fisetin to enhance the therapeutic effect of anticancer drugs. This is to exert synergistic effects on anticancer properties by simultaneously exerting the senogenic effect induced by anticancer drugs such as Dasatinib and Doxorubicin and the senolytic effect induced by chromone scaffold derivatives such as Quercetin and Fisetin on cancer cells. As for the compound according to the present invention, one compound exerts both senogenic and senolytic effects at the same time, and thus, the effect of the combination administration agent, which has been actively studied recently, can be exerted by using one compound alone (refer to FIG. 22).

Although the present invention has been described in detail with reference to the specific features, it will be apparent to those skilled in the art that this specific description is only of a preferred exemplary embodiment thereof, and does not limit the scope of the present invention. Thus, the substantial scope of the present invention will be defined by the appended claims and equivalents thereof.
CS: Chromone Scaffold
CSD: Chromone Scaffold Derivatives
NHLF: Normal Human Lung Fibroblasts
RS: Replicative senescence
OIS: Oncogene-Induced Senescence
MNC: Multinucleation
MTMP: Mitochondrial Membrane Potential
NAD: Nicotinamide Adenine Dinucleotide
NAMPT: Nicotinamide Phosphoribosyltransferase

### [Industrial Applicability]

Since the compound of the present invention can discriminate cancer cells from normal cells and induce cancer cell-specific cell senescence and apoptosis at the same time, there is no need to attach a cancer cell-specific targeting material, and it is possible to minimize side effects that often occur when anticancer drugs attack normal cells, and due to the aforementioned dual action, it is possible to exert a strong anticancer effect.

## Claims

1. A pharmaceutical composition for preventing or treating tumor or cancer disease, comprising:
a compound represented by Chemical Formula 1 below or a salt thereof as an active ingredient: 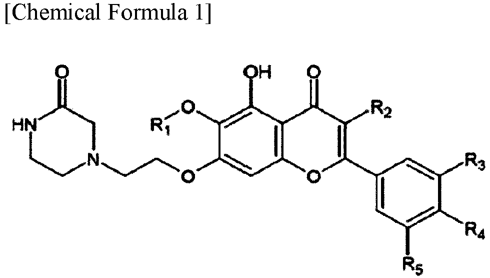
wherein in Chemical Formula 1 above,
R₁ is a C₁₋₅ alkyl, a C₅₋₆ cyclic alkyl, a C₅₋₆ cyclic alkyl containing at least one heteroatom of O or N, a C₆₋₁₂ aryl or a C₅₋₆ heteroaryl containing at least one heteroatom of O or N,
wherein R₂ is hydrogen, ethyl, acetyl, acetoxy, carboxy, benzoyloxy or 3,4,5-trihydroxybenzoyloxy,
wherein R₃ is hydrogen, hydroxy, methyl, methoxy, acetoxy, carboxy or benzoyloxy,
wherein R₄ is hydrogen, hydroxy, methyl, methoxy, acetoxy, carboxy or benzoyloxy, and
wherein R₅ is hydrogen, hydroxy, methyl, methoxy, acetoxy, carboxy or benzoyloxy.

2. The pharmaceutical composition of claim 1, wherein R₁ is methyl, ethyl, cyclopentyl, cyclohexyl or phenyl.

3. The pharmaceutical composition of claim 1, wherein R₁ is methyl,
wherein R₂ is hydrogen,
wherein R₃ is hydrogen, hydroxy or methoxy,
wherein R₄ is hydroxy or methoxy, and
wherein R₅ is hydrogen, hydroxy or methoxy.

4. The pharmaceutical composition of claim 1, wherein the compound is represented by any one of Chemical Formulas 2 to 5 below:

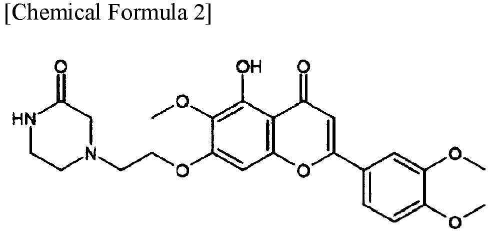

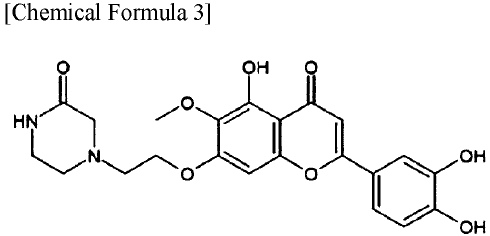

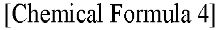

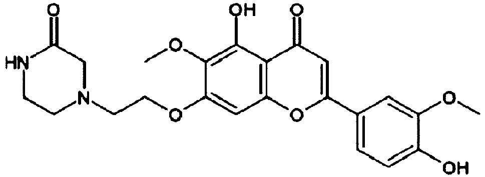

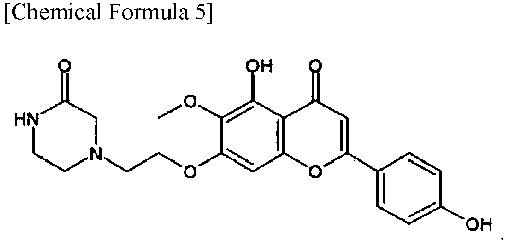

5. The pharmaceutical composition of claim 1, wherein the cancer is blood cancer or solid cancer.

6. The pharmaceutical composition of claim 5, wherein the blood cancer is leukemia, malignant lymphoma, multiple myeloma or aplastic anemia.

7. The pharmaceutical composition of claim 5, wherein the solid cancer is brain tumor, benign astrocytoma, malignant astrocytoma, pituitary adenoma, meningioma, brain lymphoma, oligodendroglioma, craniopharyngioma, ependymoma, brainstem tumor, head and neck tumor, laryngeal cancer, oropharyngeal cancer, nasal cancer, nasopharyngeal cancer, salivary gland cancer, hypopharyngeal cancer, thyroid cancer, oral cancer, chest tumor, small cell lung cancer, non-small cell lung cancer, thymus cancer, mediastinal tumor, esophageal cancer, breast cancer, male breast cancer, abdominal tumor, stomach cancer, liver cancer, gallbladder cancer, biliary tract cancer, pancreatic cancer, small intestine cancer, colon cancer, anal cancer, bladder cancer, kidney cancer, male genital tumor, penile cancer, prostate cancer, female genital tumor, cervical cancer, endometrial cancer, ovarian cancer, uterine sarcoma, vaginal cancer, female external genital cancer, female urethral cancer or skin cancer.

8. The pharmaceutical composition of claim 1, wherein the compound exhibits antitumor or anticancer activity by at least one action selected from the group consisting of:
(i) inhibition of tumor or cancer cell-specific proliferation by the induction of replicative senescence (RS) and/or oncogene-induced senescence (OIS);
(ii) reversal of the tumor or cancer cell NAD/NADH ratio;
(iii) upregulation of tumor or cancer cell ROS; and
(iv) inducing tumor or cancer cell-specific apoptosis.

9. A quasi-drug composition for preventing or ameliorating tumor or cancer disease, comprising:
a compound represented by Chemical Formula 1 below or a salt thereof as an active ingredient: 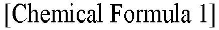 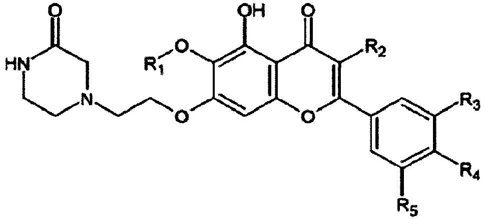
wherein in Chemical Formula 1 above,
R₁ is a C₁₋₅ alkyl, a C₅₋₆ cyclic alkyl, a C₅₋₆ cyclic alkyl containing at least one heteroatom of O or N, a C₆₋₁₂ aryl or a C₅₋₆ heteroaryl containing at least one heteroatom of O or N,
wherein R₂ is hydrogen, ethyl, acetyl, acetoxy, carboxy, benzoyloxy or 3,4,5-trihydroxybenzoyloxy,
wherein R₃ is hydrogen, hydroxy, methyl, methoxy, acetoxy, carboxy or benzoyloxy,
wherein R₄ is hydrogen, hydroxy, methyl, methoxy, acetoxy, carboxy or benzoyloxy, and
wherein R₅ is hydrogen, hydroxy, methyl, methoxy, acetoxy, carboxy or benzoyloxy.

10. The quasi-drug composition of claim 9, wherein R₁ is methyl, ethyl, cyclopentyl, cyclohexyl or phenyl.

11. The quasi-drug composition of claim 9, wherein R₁ is methyl,
wherein R₂ is hydrogen,
wherein R₃ is hydrogen, hydroxy or methoxy,
wherein R₄ is hydroxy or methoxy, and
wherein R₅ is hydrogen, hydroxy or methoxy.

12. The quasi-drug composition of claim 9, wherein the compound is represented by any one of Chemical Formulas 2 to 5 below:

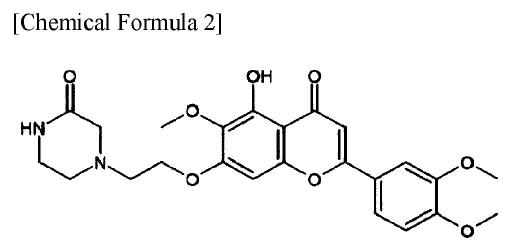

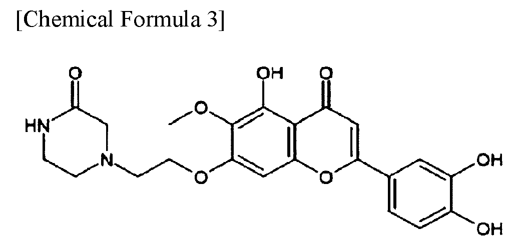

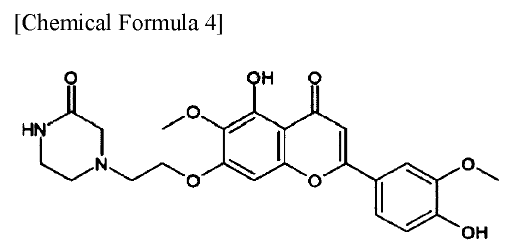

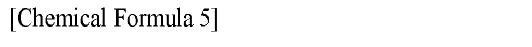

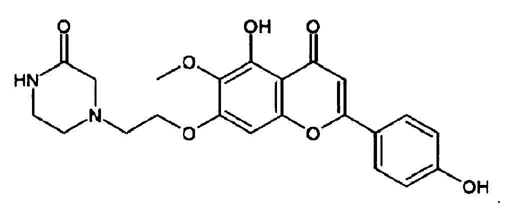

13. The quasi-drug composition of claim 9, wherein the cancer is blood cancer or solid cancer.

14. The quasi-drug composition of claim 13, wherein the blood cancer is leukemia, malignant lymphoma, multiple myeloma or aplastic anemia.

15. The quasi-drug composition of claim 13, wherein the solid cancer is brain tumor, benign astrocytoma, malignant astrocytoma, pituitary adenoma, meningioma, brain lymphoma, oligodendroglioma, craniopharyngioma, ependymoma, brainstem tumor, head and neck tumor, laryngeal cancer, oropharyngeal cancer, nasal cancer, nasopharyngeal cancer, salivary gland cancer, hypopharyngeal cancer, thyroid cancer, oral cancer, chest tumor, small cell lung cancer, non-small cell lung cancer, thymus cancer, mediastinal tumor, esophageal cancer, breast cancer, male breast cancer, abdominal tumor, stomach cancer, liver cancer, gallbladder cancer, biliary tract cancer, pancreatic cancer, small intestine cancer, colon cancer, anal cancer, bladder cancer, kidney cancer, male genital tumor, penile cancer, prostate cancer, female genital tumor, cervical cancer, endometrial cancer, ovarian cancer, uterine sarcoma, vaginal cancer, female external genital cancer, female urethral cancer or skin cancer.

16. The quasi-drug composition of claim 9, wherein the compound exhibits antitumor or anticancer activity by at least one action selected from the group consisting of:
(i) inhibition of tumor or cancer cell-specific proliferation by the induction of replicative senescence (RS) and/or oncogene-induced senescence (OIS);
(ii) reversal of the tumor or cancer cell NAD/NADH ratio;
(iii) upregulation of tumor or cancer cell ROS; and
(iv) inducing tumor or cancer cell-specific apoptosis.

17. A food composition for preventing or ameliorating tumor or cancer disease, comprising:
a compound represented by Chemical Formula 1 below or a salt thereof as an active ingredient: 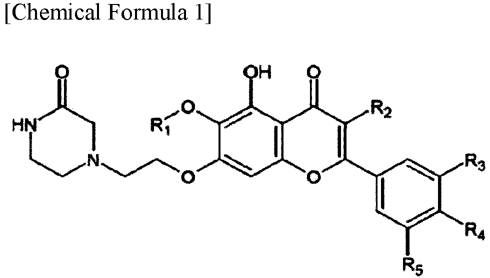
wherein in Chemical Formula 1 above,
R₁ is a C₁₋₅ alkyl, a C₅₋₆ cyclic alkyl, a C₅₋₆ cyclic alkyl containing at least one heteroatom of O or N, a C₆₋₁₂ aryl or a C₅₋₆ heteroaryl containing at least one heteroatom of O or N,
wherein R₂ is hydrogen, ethyl, acetyl, acetoxy, carboxy, benzoyloxy or 3,4,5-trihydroxybenzoyloxy,
wherein R₃ is hydrogen, hydroxy, methyl, methoxy, acetoxy, carboxy or benzoyloxy,
wherein R₄ is hydrogen, hydroxy, methyl, methoxy, acetoxy, carboxy or benzoyloxy, and
wherein R₅ is hydrogen, hydroxy, methyl, methoxy, acetoxy, carboxy or benzoyloxy.

18. The food composition of claim 17, wherein R₁ is methyl, ethyl, cyclopentyl, cyclohexyl or phenyl.

19. The food composition of claim 17, wherein R₁ is methyl,
wherein R₂ is hydrogen,
wherein R₃ is hydrogen, hydroxy or methoxy,
wherein R₄ is hydroxy or methoxy, and
wherein R₅ is hydrogen, hydroxy or methoxy.

20. The food composition of claim 17, wherein the compound is represented by any one of Chemical Formulas 2 to 5 below:

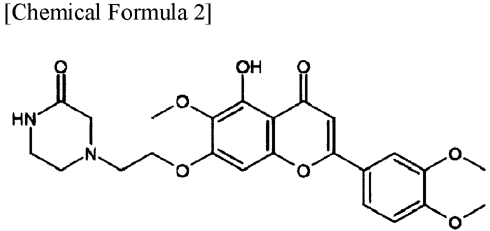

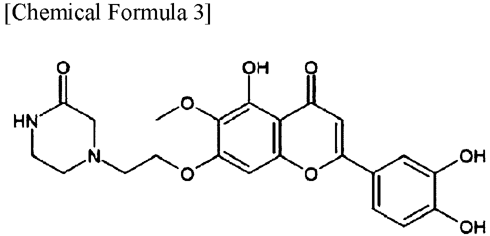

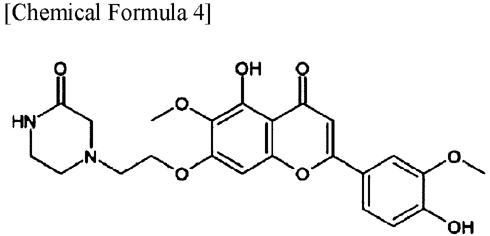

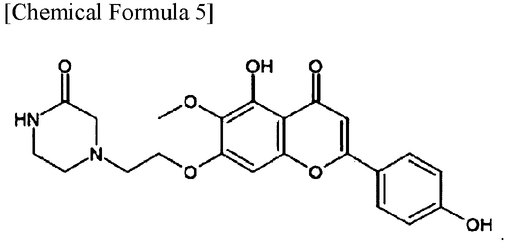

21. The food composition of claim 17, wherein the cancer is blood cancer or solid cancer.

22. The food composition of claim 21, wherein the blood cancer is leukemia, malignant lymphoma, multiple myeloma or aplastic anemia.

23. The food composition of claim 21, wherein the solid cancer is brain tumor, benign astrocytoma, malignant astrocytoma, pituitary adenoma, meningioma, brain lymphoma, oligodendroglioma, craniopharyngioma, ependymoma, brainstem tumor, head and neck tumor, laryngeal cancer, oropharyngeal cancer, nasal cancer, nasopharyngeal cancer, salivary gland cancer, hypopharyngeal cancer, thyroid cancer, oral cancer, chest tumor, small cell lung cancer, non-small cell lung cancer, thymus cancer, mediastinal tumor, esophageal cancer, breast cancer, male breast cancer, abdominal tumor, stomach cancer, liver cancer, gallbladder cancer, biliary tract cancer, pancreatic cancer, small intestine cancer, colon cancer, anal cancer, bladder cancer, kidney cancer, male genital tumor, penile cancer, prostate cancer, female genital tumor, cervical cancer, endometrial cancer, ovarian cancer, uterine sarcoma, vaginal cancer, female external genital cancer, female urethral cancer or skin cancer.

24. The food composition of claim 17, wherein the compound exhibits antitumor or anticancer activity by at least one action selected from the group consisting of:
(i) inhibition of tumor or cancer cell-specific proliferation by the induction of replicative senescence (RS) and/or oncogene-induced senescence (OIS);
(ii) reversal of the tumor or cancer cell NAD/NADH ratio;
(iii) upregulation of tumor or cancer cell ROS; and
(iv) inducing tumor or cancer cell-specific apoptosis.
